# EUROPEAN PATENT SPECIFICATION

(11) **EP 3 548 035 B1**
(45) Date of publication and mention of the grant of the patent: **21.10.2026**
(21) Application number: 17877310.7
(22) Date of filing: 30.11.2017
(51) Int. Cl.: A61K 31/56, A61K 31/4743, A61P 17/02, C07D 495/04, A61P 1/04, A61K 31/573, A61K 31/4365, A61K 45/06, A61K 31/519

(54) **COMBINATION OF A 15-PGDH INHIBITOR WITH DEXAMETHASONE FOR USE IN TREATING INTESTINAL OR GASTROINTESTINAL DISORDERS**
KOMBINATION VON EINEM 15-PGDH-INHIBITOR MIT DEXAMETHASONE ZUR VERWENDUNG BEI DER BEHANDLUNG VON DARM- ODER MAGEN-DARM-ERKRANKUNGEN
COMBINAISON D'UN INHIBITEUR DE 15-PGDH AVEC DE LA DEXAMÉTHASONE POUR SON UTILISATION DANS LE TRAITEMENT DES TROUBLES INTESTINAUX OU GASTRO-INTESTINAUX

(30) Priority: 30.11.2016 US 201662428259 P; 23.05.2017 US 201762510166 P
(43) Date of publication of application: 09.10.2019
(73) Proprietor: Case Western Reserve University, Cleveland, OH 44106 (US)
(72) Inventor: MARKOWITZ, Sanford, Cleveland, Ohio 44106 (US); HO, Won Jin, Cleveland, Ohio 44106 (US); FINK, Stephen P., Cleveland, Ohio 44106 (US); VARADAN, Vinay, Cleveland, Ohio 44106 (US)
(74) Representative: Maiwald GmbH
(86) International application number: PCT/US2017/063959
(87) International publication number: WO 2018/102552

(56) References cited:
- WO-A1-2004/099204
- WO-A1-2010/023181
- WO-A1-2015/065716
- WO-A1-2018/218251
- WO-A1-90/06100
- US-A- 5 405 842
- US-A1- 2006 233 797
- US-A1- 2006 287 284
- US-A1- 2007 059 265
- US-A1- 2010 120 732
- US-A1- 2011 009 374
- US-A1- 2011 195 031
- US-A1- 2015 118 744
- US-A1- 2016 136 185
- TONG, M ET AL.: "15-Hydroxyprostaglandin dehydrogenase can be induced by dexamethasone and other glucocorticoids at the therapeutic level in A549 human lung adenocarcinoma cells", ARCHIVES OF BIOCHEMISTRY AND BIOPHYSICS, vol. 435, no. 1, 2005, pages 50 - 55, XP055509222

## Description

### CROSS-REFERENCE TO RELATED APPLICATIONS

This application claims priority to U.S. Provisional Application No. 62/428,259, filed on November 30, 2016, and U.S. Provisional Application No. 62/510,166, filed on May 23, 2017.

### GOVERNMENT FUNDING

This invention was made with government support under Grant No. DK150964 AND CA150964, awarded by The National Institutes of Health. The United States government has certain rights in the invention.

### BACKGROUND

Short-chain dehydrogenases (SCDs) are a family of dehydrogenases that share only 15% to 30% sequence identity, with similarity predominantly in the coenzyme binding domain and the substrate binding domain. In addition to their role in detoxification of ethanol, SCDs are involved in synthesis and degradation of fatty acids, steroids, and some prostaglandins, and are therefore implicated in a variety of disorders, such as lipid storage disease, myopathy, SCD deficiency, and certain genetic disorders.

The SCD, 15-hydroxy-prostaglandin dehydrogenase (15-PGDH), (hydroxyprostaglandin dehydrogenase 15-(nicotinamide adeninedinucleotide); 15-PGDH; Enzyme Commission number 1.1.1.141; encoded by the HPGD gene), represents the key enzyme in the inactivation of a number of active prostaglandins, leukotrienes and hydroxyeicosatetraenoic acids (HETEs) (*e.g.,* by catalyzing oxidation of PGE₂ to 15-keto-prostaglandin E2, 15k-PGE). The human enzyme is encoded by the HPGD gene and consists of a homodimer with subunits of a size of 29 kDa. The enzyme belongs to the evolutionarily conserved superfamily of short-chain dehydrogenase/reductase enzymes (SDRs), and according to the recently approved nomenclature for human enzymes, it is named SDR36C1. Thus far, two forms of 15-PGDH enzyme activity have been identified, NAD+-dependent type I 15-PGDH, which is encoded by the HPGD gene, and the type II NADP-dependent 15-PGDH, also known as carbonyl reductase 1 (CBR1, SDR21C1). However, the preference of CBR1 for NADP and the high Km values of CBR1 for most prostaglandin suggest that the majority of the *in vivo* activity can be attributed to type I 15-PGDH encoded by the HPGD gene, that hereafter, and throughout all following text, simply denoted as 15-PGDH. WO 2015/065716 A1 relates to compositions and methods of modulating short-chain dehydrogenase activity.

### SUMMARY

The invention is set out in the appended set of claims. The references to the methods of treatment by therapy or surgery or *in vivo* diagnosis methods in the examples of the description are to be interpreted as references to compounds, pharmaceutical compositions and medicaments of the present invention for use in those methods.

In the present invention, an inflammatory and/or immune disease or disorder treated with the combination of 15-PGDH inhibitor and a corticosteroid includes intestinal, gastrointestinal, or bowel disorders. As described below, it was found that inhibitors of short-chain dehydrogenase activity, such as 15-PGDH inhibitors, can be administered to a subject in need thereof in combination with corticosteroids to treat intestinal, gastrointestinal, or bowel disorders, such as oral ulcers, gum disease, gastritis, colitis, ulcerative colitis, gastric ulcers, inflammatory bowel disease, and Crohn's disease.

### BRIEF DESCRIPTION OF THE DRAWINGS

Fig. 1 illustrates a graph showing the average changes from baseline weight of the cohort of control (diamonds) versus SW033291 (squares) treated mice all treated with 2% dextran sulfate sodium (DSS) in the drinking water.
Fig. 2 illustrates a graph of the daily disease activity index of the cohort of control (diamonds) versus SW033291 (squares) treated mice all treated with 2% DSS in the drinking water.
Fig. 3 illustrates a graph showing the average changes from baseline weight of the cohort of DSS treated mice receiving a control vehicle (diamonds) versus SW033291 (squares).
Figs. 4(A-B) illustrate: (A) a graph showing the number of ulcers in a colon of DSS treated mice receiving a control vehicle versus SW033291; and (B) photographs showing ulcers of DSS treated mice receiving control (left) or SW033291 (right).
Fig. 5 illustrates a graph showing quantitation of ulcer burden on day 15 of DSS treated mice receiving a control vehicle or SW033291.
Figs. 6(A-B) illustrate photographs showing colonoscopic findings and mouse endoscopic index of colitis severity (MEICs) for a DSS treated mouse receiving a control vehicle or SW033291.
Fig. 7 illustrates a graph showing MEICS score of DSS treated mice receiving a control vehicle or SW033291.
Fig. 8 illustrates photomicrographs of high powered fields from the mid-colon on day 8 of the DSS protocol from control mice, SW033291 treated mice (treatment) and 15-PGDHknockout mice (KO) and a graph depicting sum of the average number of BrdU positive cells per crypt in the distal plus middle colons of control (Cn), SW033219 treated mice (Tx), and 15-PGDH knockout mice (KO) on day 1, day 8, and day 15 of the DSS treatment protocol.
Fig. 9 illustrates a graph showing colon length at day 22 of DSS treated mice receiving a control vehicle or SW033291.
Fig. 10 is a schematic illustration showing PARADIGM SuperPathway sub-networks whose activities are significantly correlated with 15-PGDH gene expression in normal colon tissues.
Figs. 11(A-C) illustrate: (A) a schema of a study in which mice received three daily doses of dexamethasone and were sacrificed 6 hours after the third dose for analysis; (B) representative western blot analysis showing dexamethasone induction of 15-PGDH protein in mouse colon, at two different doses of dexamethasone; and (C) graphical summary of real time RT-PCR from all mice in the study showing an approximate doubling of colon 15-PGDH expression level by dexamethasone treatment.
Figs. 12(A-B) illustrate: (A) a schema of a study in which mice received three daily doses of dexamethasone and were sacrificed 6 hours after the third dose for analysis; and (B) a graph showing near doubling of 15-PGDH enzyme activity in colons of dexamethasone treated mice.
Figs. 13(A-B) illustrate graphs showing higher dexamethasone doses exacerbate colitis induction by DSS.
Fig. 14 illustrate a schema of a study in which mice receive 7 days of 2.5% DSS in drinking water (from day 1 to day 8), a regime that induces murine colitis, and followed by treatment with vehicle, (+) SW033291, dexamethasone, or both (+) SW033291 and dexamethasone.
Fig. 15 illustrates plots showing daily weights of mice on the study from days 1-17 in mice administered (+) SW033291 and dexamethasone treatment individually or in combination.
Fig. 16 illustrates plots of disease activity (DAI) as measured by the disease activity index in which diarrhea (on a 0-3 scale) and fecal blood (on a 0-3 scale) are combined (on a 0-6 scale) in mice administered (+) SW033291 and dexamethasone treatment individually or in combination.
Figs. 17(A-B) illustrate graphs showing area under the DAI curve (total DAI) at left, and showing the percent decrease in total DAI (relative disease reduction) graph at right of the results of Fig. 16.
Fig. 18 illustrates a graph showing the survival of mice on a daily basis for each treatment arm through day 16 of the disease model.
Fig. 19 illustrates a graph of data shown in Fig. 17B with the addition of p values and reordering of arms.
Figs. 20(A-D) show representative endoscopic image for each treatment group on day 13 of treatment.
Fig. 21 illustrates a graph showing murine endoscopic index of colitis severity (MEICS) scores on day 13 for each treatment group. **p<0.01, ***p<0.005 by ANOVA and Student's t-test.
Figs. 22A-D show representative histological pictures of distal colons on day 13 of each treatment group (A) control, (B) dexamethasone, (C) SW033291, and (D) combination.
Fig. 23 graphs semi-quantitatively scored histological extent of inflammatory damage to the crypts.
Fig. 24 graphs the severity of mesenteric lymphadenopathy assessed by collective mesenteric lymph node weight normalized by body weight on day 13 of each treatment group.

### DETAILED DESCRIPTION

For convenience, certain terms employed in the specification, examples, and appended claims are collected here. Unless defined otherwise, all technical and scientific terms used herein have the same meaning as commonly understood by one of ordinary skill in the art to which this application belongs.

The articles "a" and "an" are used herein to refer to one or to more than one (*i.e.,* to at least one) of the grammatical object of the article. By way of example, "an element" means one element or more than one element.

The terms "comprise," "comprising," "include," "including," "have," and "having" are used in the inclusive, open sense, meaning that additional elements may be included. The terms "such as", "*e.g.*", as used herein are non-limiting and are for illustrative purposes only. "Including" and "including but not limited to" are used interchangeably.

The term "or" as used herein should be understood to mean "and/or", unless the context clearly indicates otherwise.

As used herein, the term "about" or "approximately" refers to a quantity, level, value, number, frequency, percentage, dimension, size, amount, weight or length that varies by as much as 15%, 10%, 9%, 8%, 7%, 6%, 5%, 4%, 3%, 2% or 1% to a reference quantity, level, value, number, frequency, percentage, dimension, size, amount, weight or length. In one embodiment, the term "about" or "approximately" refers a range of quantity, level, value, number, frequency, percentage, dimension, size, amount, weight or length ± 15%, ± 10%, ± 9%, ± 8%, ± 7%, ± 6%, ± 5%, ± 4%, ± 3%, ± 2%, or ± 1% about a reference quantity, level, value, number, frequency, percentage, dimension, size, amount, weight or length.

It will be noted that the structure of some of the compounds of the application include asymmetric (chiral) carbon or sulfur atoms. It is to be understood accordingly that the isomers arising from such asymmetry are included herein, unless indicated otherwise. Such isomers can be obtained in substantially pure form by classical separation techniques and by stereochemically controlled synthesis. The compounds of this application may exist in stereoisomeric form, therefore can be produced as individual stereoisomers or as mixtures.

The term "isomerism" means compounds that have identical molecular formulae but that differ in the nature or the sequence of bonding of their atoms or in the arrangement of their atoms in space. Isomers that differ in the arrangement of their atoms in space are termed "stereoisomers". Stereoisomers that are not mirror images of one another are termed "diastereoisomers", and stereoisomers that are non-superimposable mirror images are termed "enantiomers", or sometimes optical isomers. A carbon atom bonded to four nonidentical substituents is termed a "chiral center" whereas a sulfur bound to three or four different substitutents, e.g., sulfoxides or sulfinimides, is likewise termed a "chiral center".

The term "chiral isomer" means a compound with at least one chiral center. It has two enantiomeric forms of opposite chirality and may exist either as an individual enantiomer or as a mixture of enantiomers. A mixture containing equal amounts of individual enantiomeric forms of opposite chirality is termed a "racemic mixture". A compound that has more than one chiral center has 2n-1 enantiomeric pairs, where n is the number of chiral centers. Compounds with more than one chiral center may exist as either an individual diastereomer or as a mixture of diastereomers, termed a "diastereomeric mixture". When one chiral center is present, a stereoisomer may be characterized by the absolute configuration (R or S) of that chiral center. Alternatively, when one or more chiral centers are present, a stereoisomer may be characterized as (+) or (-). Absolute configuration refers to the arrangement in space of the substituents attached to the chiral center. The substituents attached to the chiral center under consideration are ranked in accordance with the Sequence Rule of Cahn, Ingold and Prelog. (Cahn et al, Angew. Chem. Inter. Edit. 1966, 5, 385; errata 511; Cahn et al., Angew. Chem. 1966, 78, 413; Cahn and Ingold, J Chem. Soc. 1951 (London), 612; Cahn et al., Experientia 1956, 12, 81; Cahn, J., Chem. Educ. 1964, 41, 116).

The term "geometric Isomers" means the diastereomers that owe their existence to hindered rotation about double bonds. These configurations are differentiated in their names by the prefixes *cis* and trans, or Z and E, which indicate that the groups are on the same or opposite side of the double bond in the molecule according to the Cahn-Ingold-Prelog rules. Further, the structures and other compounds discussed in this application include all atropic isomers thereof.

The term "atropic isomers" are a type of stereoisomer in which the atoms of two isomers are arranged differently in space. Atropic isomers owe their existence to a restricted rotation caused by hindrance of rotation of large groups about a central bond. Such atropic isomers typically exist as a mixture, however as a result of recent advances in chromatography techniques, it has been possible to separate mixtures of two atropic isomers in select cases.

The terms "crystal polymorphs" or "polymorphs" or "crystal forms" means crystal structures in which a compound (or salt or solvate thereof) can crystallize in different crystal packing arrangements, all of which have the same elemental composition. Different crystal forms usually have different X-ray diffraction patterns, infrared spectral, melting points, density hardness, crystal shape, optical and electrical properties, stability and solubility. Recrystallization solvent, rate of crystallization, storage temperature, and other factors may cause one crystal form to dominate. Crystal polymorphs of the compounds can be prepared by crystallization under different conditions.

The term "derivative" refers to compounds that have a common core structure, and are substituted with various groups as described herein.

The term "bioisostere" refers to a compound resulting from the exchange of an atom or of a group of atoms with another, broadly similar, atom or group of atoms. The objective of a bioisosteric replacement is to create a new compound with similar biological properties to the parent compound. The bioisosteric replacement may be physicochemically or topologically based. Examples of carboxylic acid bioisosteres include acyl sulfonimides, tetrazoles, sulfonates, and phosphonates. See, *e.g.,* Patani and LaVoie, Chem. Rev. 96, 3147-3176 (1996).

The phrases "parenteral administration" and "administered parenterally" are art-recognized terms, and include modes of administration other than enteral and topical administration, such as injections, and include, without limitation, intravenous, intramuscular, intrapleural, intravascular, intrapericardial, intraarterial, intrathecal, intracapsular, intraorbital, intracardiac, intradermal, intraperitoneal, transtracheal, subcutaneous, subcuticular, intra-articular, subcapsular, subarachnoid, intraspinal and intrastemal injection and infusion.

The term "treating" is art-recognized and includes inhibiting a disease, disorder or condition in a subject, *e.g*., impeding its progress; and relieving the disease, disorder or condition, *e.g*., causing regression of the disease, disorder and/or condition. Treating the disease or condition includes ameliorating at least one symptom of the particular disease or condition, even if the underlying pathophysiology is not affected.

The term "preventing" is art-recognized and includes stopping a disease, disorder or condition from occurring in a subject, which may be predisposed to the disease, disorder and/or condition but has not yet been diagnosed as having it. Preventing a condition related to a disease includes stopping the condition from occurring after the disease has been diagnosed but before the condition has been diagnosed.

The term "pharmaceutical composition" refers to a formulation containing the disclosed compounds in a form suitable for administration to a subject. In a preferred embodiment, the pharmaceutical composition is in bulk or in unit dosage form. The unit dosage form is any of a variety of forms, including, for example, a capsule, an IV bag, a tablet, a single pump on an aerosol inhaler, or a vial. The quantity of active ingredient (*e.g*., a formulation of the disclosed compound or salts thereof) in a unit dose of composition is an effective amount and is varied according to the particular treatment involved. One skilled in the art will appreciate that it is sometimes necessary to make routine variations to the dosage depending on the age and condition of the patient. The dosage will also depend on the route of administration. A variety of routes are contemplated, including oral, pulmonary, rectal, parenteral, transdermal, subcutaneous, intravenous, intramuscular, intraperitoneal, intranasal, inhalational, and the like. Dosage forms for the topical or transdermal administration of a compound described herein includes powders, sprays, ointments, pastes, creams, lotions, gels, solutions, patches, nebulized compounds, and inhalants. In a preferred embodiment, the active compound is mixed under sterile conditions with a pharmaceutically acceptable carrier, and with any preservatives, buffers, or propellants that are required.

The term "flash dose" refers to compound formulations that are rapidly dispersing dosage forms.

The term "immediate release" is defined as a release of compound from a dosage form in a relatively brief period of time, generally up to about 60 minutes. The term "modified release" is defined to include delayed release, extended release, and pulsed release. The term "pulsed release" is defined as a series of releases of drug from a dosage form. The term "sustained release" or "extended release" is defined as continuous release of a compound from a dosage form over a prolonged period.

The phrase "pharmaceutically acceptable" is art-recognized. In certain embodiments, the term includes compositions, polymers and other materials and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" is art-recognized, and includes, for example, pharmaceutically acceptable materials, compositions or vehicles, such as a liquid or solid filler, diluent, excipient, solvent or encapsulating material, involved in carrying or transporting any subject composition from one organ, or portion of the body, to another organ, or portion of the body. Each carrier must be "acceptable" in the sense of being compatible with the other ingredients of a subject composition and not injurious to the patient. In certain embodiments, a pharmaceutically acceptable carrier is non-pyrogenic. Some examples of materials which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, sunflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The compounds of the application are capable of further forming salts. All of these forms are also contemplated herein.

"Pharmaceutically acceptable salt" of a compound means a salt that is pharmaceutically acceptable and that possesses the desired pharmacological activity of the parent compound. For example, the salt can be an acid addition salt. One embodiment of an acid addition salt is a hydrochloride salt. The pharmaceutically acceptable salts can be synthesized from a parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile being preferred. Lists of salts are found in Remington's Pharmaceutical Sciences, 18th ed. (Mack Publishing Company, 1990).

The compounds described herein can also be prepared as esters, for example pharmaceutically acceptable esters. For example, a carboxylic acid function group in a compound can be converted to its corresponding ester, *e.g.,* a methyl, ethyl, or other ester. Also, an alcohol group in a compound can be converted to its corresponding ester, *e.g.,* an acetate, propionate, or other ester.

The compounds described herein can also be prepared as prodrugs, for example pharmaceutically acceptable prodrugs. The terms "pro-drug" and "prodrug" are used interchangeably herein and refer to any compound, which releases an active parent drug *in vivo*. Since prodrugs are known to enhance numerous desirable qualities of pharmaceuticals (*e.g*., solubility, bioavailability, manufacturing, etc.) the compounds can be delivered in prodrug form. Thus, the compounds described herein are intended to cover prodrugs of the presently claimed compounds, methods of delivering the same and compositions containing the same. "Prodrugs" are intended to include any covalently bonded carriers that release an active parent drug *in vivo* when such prodrug is administered to a subject. Prodrugs are prepared by modifying functional groups present in the compound in such a way that the modifications are cleaved, either in routine manipulation or *in vivo,* to the parent compound. Prodrugs include compounds wherein a hydroxy, amino, sulfhydryl, carboxy, or carbonyl group is bonded to any group that may be cleaved *in vivo* to form a free hydroxyl, free amino, free sulfhydryl, free carboxy or free carbonyl group, respectively. Prodrugs can also include a precursor (forerunner) of a compound described herein that undergoes chemical conversion by metabolic processes before becoming an active or more active pharmacological agent or active compound described herein.

Examples of prodrugs include, but are not limited to, esters (*e.g.*, acetate, dialkylaminoacetates, formates, phosphates, sulfates, and benzoate derivatives) and carbamates (*e.g*., N,N-dimethylaminocarbonyl) of hydroxy functional groups, ester groups (*e.g.,* ethyl esters, morpholinoethanol esters) of carboxyl functional groups, N-acyl derivatives (*e.g*., N-acetyl) N-Mannich bases, Schiff bases and enaminones of amino functional groups, oximes, acetals, ketals and enol esters of ketone and aldehyde functional groups in compounds, and the like, as well as sulfides that are oxidized to form sulfoxides or sulfones.

The term "protecting group" refers to a grouping of atoms that when attached to a reactive group in a molecule masks, reduces or prevents that reactivity. Examples of protecting groups can be found in Green and Wuts, Protective Groups in Organic Chemistry, (Wiley, 2.sup.nd ed. 1991); Harrison and Harrison et al., Compendium of Synthetic Organic Methods, Vols. 1-8 (John Wiley and Sons, 1971-1996); and Kocienski, Protecting Groups, (Verlag, 3rd ed. 2003).

The term "amine protecting group" is intended to mean a functional group that converts an amine, amide, or other nitrogen-containing moiety into a different chemical group that is substantially inert to the conditions of a particular chemical reaction. Amine protecting groups are preferably removed easily and selectively in good yield under conditions that do not affect other functional groups of the molecule. Examples of amine protecting groups include, but are not limited to, formyl, acetyl, benzyl, t-butyldimethylsilyl, t-butyldiphenylsilyl, t-butyloxycarbonyl (Boc), p-methoxybenzyl, methoxymethyl, tosyl, trifluoroacetyl, trimethylsilyl (TMS), fluorenyl-methyloxycarbonyl, 2-trimethylsilyl-ethyoxycarbonyl, 1-methyl-1-(4-biphenylyl) ethoxycarbonyl, allyloxycarbonyl, benzyloxycarbonyl (CBZ), 2-trimethylsilyl-ethanesulfonyl (SES), trityl and substituted trityl groups, 9-fluorenylmethyloxycarbonyl (FMOC), nitro-veratryloxycarbonyl (NVOC), and the like. Those of skill in the art can identify other suitable amine protecting groups.

Representative hydroxy protecting groups include those where the hydroxy group is either acylated or alkylated such as benzyl, and trityl ethers as well as alkyl ethers, tetrahydropyranyl ethers, trialkylsilyl ethers and allyl ethers.

Additionally, the salts of the compounds described herein, can exist in either hydrated or unhydrated (the anhydrous) form or as solvates with other solvent molecules. Non-limiting examples of hydrates include monohydrates, dihydrates, etc. Nonlimiting examples of solvates include ethanol solvates, acetone solvates, etc.

The term "solvates" means solvent addition forms that contain either stoichiometric or non-stoichiometric amounts of solvent. Some compounds have a tendency to trap a fixed molar ratio of solvent molecules in the crystalline solid state, thus forming a solvate. If the solvent is water the solvate formed is a hydrate, when the solvent is alcohol, the solvate formed is an alcoholate. Hydrates are formed by the combination of one or more molecules of water with one of the substances in which the water retains its molecular state as H₂O, such combination being able to form one or more hydrate.

The compounds, salts and prodrugs described herein can exist in several tautomeric forms, including the enol and imine form, and the keto and enamine form and geometric isomers and mixtures thereof. Tautomers exist as mixtures of a tautomeric set in solution. In solid form, usually one tautomer predominates. Even though one tautomer may be described, the present application includes all tautomers of the present compounds. A tautomer is one of two or more structural isomers that exist in equilibrium and are readily converted from one isomeric form to another. This reaction results in the formal migration of a hydrogen atom accompanied by a switch of adjacent conjugated double bonds. In solutions where tautomerization is possible, a chemical equilibrium of the tautomers will be reached. The exact ratio of the tautomers depends on several factors, including temperature, solvent, and pH. The concept of tautomers that are interconvertable by tautomerizations is called tautomerism.

Of the various types of tautomerism that are possible, two are commonly observed. In keto-enol tautomerism a simultaneous shift of electrons and a hydrogen atom occurs.

Tautomerizations can be catalyzed by: Base: 1. deprotonation; 2. formation of a delocalized anion (*e.g.,* an enolate); 3. protonation at a different position of the anion; Acid: 1. protonation; 2. formation of a delocalized cation; 3. deprotonation at a different position adjacent to the cation.

The term "analogue" refers to a chemical compound that is structurally similar to another but differs slightly in composition (as in the replacement of one atom by an atom of a different element or in the presence of a particular functional group, or the replacement of one functional group by another functional group). Thus, an analogue is a compound that is similar or comparable in function and appearance, but not in structure or origin to the reference compound.

A "patient," "subject," or "host" to be treated by the subject method may mean either a human or non-human animal, such as a mammal, a fish, a bird, a reptile, or an amphibian. Thus, the subject of the herein disclosed methods can be a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered. In one aspect, the subject is a mammal. A patient refers to a subject afflicted with a disease or disorder.

The terms "prophylactic" or "therapeutic" treatment is art-recognized and includes administration to the host of one or more of the subject compositions. If it is administered prior to clinical manifestation of the unwanted condition (*e.g*., disease or other unwanted state of the host animal) then the treatment is prophylactic, *i.e.,* it protects the host against developing the unwanted condition, whereas if it is administered after manifestation of the unwanted condition, the treatment is therapeutic (*i.e*., it is intended to diminish, ameliorate, or stabilize the existing unwanted condition or side effects thereof).

The terms "therapeutic agent", "drug", "medicament" and "bioactive substance" are art-recognized and include molecules and other agents that are biologically, physiologically, or pharmacologically active substances that act locally or systemically in a patient or subject to treat a disease or condition. The terms include without limitation pharmaceutically acceptable salts thereof and prodrugs. Such agents may be acidic, basic, or salts; they may be neutral molecules, polar molecules, or molecular complexes capable of hydrogen bonding; they may be prodrugs in the form of ethers, esters, amides and the like that are biologically activated when administered into a patient or subject.

The phrase "therapeutically effective amount" or "pharmaceutically effective amount" is an art-recognized term. In certain embodiments, the term refers to an amount of a therapeutic agent that produces some desired effect at a reasonable benefit/risk ratio applicable to any medical treatment. In certain embodiments, the term refers to that amount necessary or sufficient to eliminate, reduce or maintain a target of a particular therapeutic regimen. The effective amount may vary depending on such factors as the disease or condition being treated, the particular targeted constructs being administered, the size of the subject or the severity of the disease or condition. One of ordinary skill in the art may empirically determine the effective amount of a particular compound without necessitating undue experimentation. In certain embodiments, a therapeutically effective amount of a therapeutic agent for *in vivo* use will likely depend on a number of factors, including: the rate of release of an agent from a polymer matrix, which will depend in part on the chemical and physical characteristics of the polymer; the identity of the agent; the mode and method of administration; and any other materials incorporated in the polymer matrix in addition to the agent.

The term "ED50" is art-recognized. In certain embodiments, ED50 means the dose of a drug, which produces 50% of its maximum response or effect, or alternatively, the dose, which produces a pre-determined response in 50% of test subjects or preparations. The term "LD50" is art-recognized. In certain embodiments, LD50 means the dose of a drug, which is lethal in 50% of test subjects. The term "therapeutic index" is an art-recognized term, which refers to the therapeutic index of a drug, defined as LD50/ED50.

The terms "IC₅₀," or "half maximal inhibitory concentration" is intended to refer to the concentration of a substance (*e.g*., a compound or a drug) that is required for 50% inhibition of a biological process, or component of a process, including a protein, subunit, organelle, ribonucleoprotein, etc.

With respect to any chemical compounds, the present application is intended to include all isotopes of atoms occurring in the present compounds. Isotopes include those atoms having the same atomic number but different mass numbers. By way of general example and without limitation, isotopes of hydrogen include tritium and deuterium, and isotopes of carbon include C-13 and C-14.

When a bond to a substituent is shown to cross a bond connecting two atoms in a ring, then such substituent can be bonded to any atom in the ring. When a substituent is listed without indicating the atom via which such substituent is bonded to the rest of the compound of a given formula, then such substituent can be bonded via any atom in such substituent. Combinations of substituents and/or variables are permissible, but only if such combinations result in stable compounds.

The terms "stable compound" and "stable structure" are meant to indicate a compound that is sufficiently robust to survive isolation, and as appropriate, purification from a reaction mixture, and formulation into an efficacious therapeutic agent.

The terms "free compound" is used herein to describe a compound in the unbound state.

Throughout the description, where compositions are described as having, including, or comprising, specific components, it is contemplated that compositions also consist essentially of, or consist of, the recited components. Similarly, where methods or processes are described as having, including, or comprising specific process steps, the processes also consist essentially of, or consist of, the recited processing steps. Further, it should be understood that the order of steps or order for performing certain actions is immaterial so long as the compositions and methods described herein remains operable. Moreover, two or more steps or actions can be conducted simultaneously.

The term "small molecule" is an art-recognized term. In certain embodiments, this term refers to a molecule, which has a molecular weight of less than about 2000 amu, or less than about 1000 amu, and even less than about 500 amu.

All percentages and ratios used herein, unless otherwise indicated, are by weight.

The terms "gene expression" or "protein expression" includes any information pertaining to the amount of gene transcript or protein present in a sample, as well as information about the rate at which genes or proteins are produced or are accumulating or being degraded (*e.g*., reporter gene data, data from nuclear runoff experiments, pulse-chase data etc.). Certain kinds of data might be viewed as relating to both gene and protein expression. For example, protein levels in a cell are reflective of the level of protein as well as the level of transcription, and such data is intended to be included by the phrase "gene or protein expression information". Such information may be given in the form of amounts per cell, amounts relative to a control gene or protein, in unitless measures, etc.; the term "information" is not to be limited to any particular means of representation and is intended to mean any representation that provides relevant information. The term "expression levels" refers to a quantity reflected in or derivable from the gene or protein expression data, whether the data is directed to gene transcript accumulation or protein accumulation or protein synthesis rates, etc.

The terms "healthy" and "normal" are used interchangeably herein to refer to a subject or particular cell or tissue that is devoid (at least to the limit of detection) of a disease condition.

The term "nucleic acid" refers to polynucleotides such as deoxyribonucleic acid (DNA), and, where appropriate, ribonucleic acid (RNA). The term should also be understood to include analogues of either RNA or DNA made from nucleotide analogues, and, as applicable to the embodiment being described, single-stranded (such as sense or antisense) and double-stranded polynucleotides. In some embodiments, "nucleic acid" refers to inhibitory nucleic acids. Some categories of inhibitory nucleic acid compounds include antisense nucleic acids, RNAi constructs, and catalytic nucleic acid constructs. Such categories of nucleic acids are well-known in the art.

The term "corticosteroid resistance to the anti-inflammatory effects of corticosteroids" refers to no clinical improvement after treatment with high-dose glucocorticoid.

The term "corticosteroid dependence" refers to a condition that initially responds to corticosteroids but relapses quickly upon drug withdrawal or dose tapering.

The term "corticosteroid refractory response" refers to a condition that does not respond to an adequate induction dose of corticosteroids. It includes relatively or totally refractory responses to glucocorticoid therapy, and often needs to be controlled by add-on treatment.

Other types of corticosteroid ineffectiveness include the need for a very high dose treatment, "difficult to treat" and "do not respond well" or severe cases, and impaired in vitro and in vivo responsiveness.

The term "corticosteroid intolerance" refers to toxicity of the therapy and/or risks for developing corticosteroid-related adverse events such as opportunistic infections and bone loss.
Embodiments described herein relate to the use of 15-PGDH inhibitors in combination with corticosteroids to treat inflammation and/or reduce aberrant activity of the immune system in a subject in need thereof. It was found that corticosteroids administered to a subject can induce 15-PGDH expression in tissue of the subject. Administration of a 15-PGDH inhibitor in combination with a corticosteroid was found to enhance anti-inflammatory and/or immunosuppressive effects of the corticosteroid while attenuating corticosteroid induced adverse and/or cytotoxic effects. Treatment of inflammatory and/or immune disorders by administration of 15-PGDH inhibitors in combination with corticosteroids can increase therapeutic efficacy and can allow the corticosteroids to be administered, in some instances, at lower dosages to achieve similar effects, and, in other instances, at higher dosages and for prolonged periods of times with attenuated and/or reduced adverse or cytotoxic effects.

The 15-PGDH inhibitors are administered in combination with corticosteroids to treat intestinal, gastrointestinal, or bowel disorders. The intestinal, gastrointestinal, or bowel disorders treated can include oral ulcers, gum disease, gastritis, colitis, ulcerative colitis, gastric ulcers, inflammatory bowel disease, and Crohn's disease. As described below, it was found that inhibitors of short-chain dehydrogenase activity, such as 15-PGDH inhibitors, are administered to a subject in need thereof in combination with corticosteroids to treat intestinal, gastrointestinal, or bowel disorders, such as oral ulcers, gum disease, gastritis, colitis, ulcerative colitis, gastric ulcers, inflammatory bowel disease, and Crohn's disease.

The 15-PGDH inhibitors described herein are used in a pharmaceutical composition for the prevention or the treatment of oral, intestinal, and/or gastrointestinal injury or diseases, or inflammatory bowel disease (IBD), such as Crohn's disease, oral ulcers, gum disease, gastritis, colitis, ulcerative colitis, and gastric ulcers. Gastritis and gastric ulcer, representatives of the gastrointestinal diseases, are defined as the conditions where gastrointestinal mucus membrane is digested by gastric acid to form ulcer. In the stomach walls generally consisting of mucosa, submucosa, muscle layer and serosa, gastric ulcer even damages submucosa and muscle layer, while gastritis damages mucosa only. Although the morbidity rates of gastritis and gastric ulcer are relatively high, the causes thereof have not been clarified yet. Until now, they are known to be caused by an imbalance between aggressive factors and defensive factors, that is, the increase in aggressive factors such as the increase in gastric acid or pepsin secretion, or the decrease in defensive factors such as structural or morphological deficit of the gastric mucus membrane, the decrease in mucus and bicarbonate ion secretion, the decrease in prostaglandin production, or the like.

Currently available therapeutic agents for gastritis and gastric ulcer comprise various drugs for strengthening the defensive factors such as an antacid, which does not affect, gastric acid secretion but neutralizes gastric acid that has been already produced, an inhibitor of gastric acid secretion, a promoter of prostaglandin secretion, and a coating agent for stomach walls. Especially, prostaglandins are known to be essential in maintaining the mechanism for protecting and defending gastric mucus membrane (Wallace J L., 2008, Physiol Rev., 88(4), 1547-65, S. J. Konturek et al., 2005, Journal of Physiology and Pharmacology, 56(5)). In view of the above, since the 15-PGDH inhibitors described herein show a suppressive or inhibitory activity against 15-PGDH, which degrades prostaglandins that protect gastric mucus membrane, they can be effective for the prevention or the treatment of gastrointestinal diseases, inter alia, gastritis and gastric ulcer.

Additionally, corticosteroids and TNF alpha antagonists are both used in the treatment of ulcerative colitis and IBD patients. In mouse models, 15-PGDH inhibitors speed healing of ulcerative colitis. We have found that administering corticosteroids to mice elevates levels of colon 15-PGDH, an effect that should reduce the therapeutic effectiveness of corticosteroids in colitis treatment. This suggests that combining a corticosteroid with a 15-PGDH inhibitor should be more effective in colitis (and IBD) treatment than using either agent alone.

Similarly, we have comparatively shown that TNF-alpha suppresses colon 15-PGDH expression. This suggests that TNF-alpha antagonists will increase colon 15-PGDH expression, an effect that should reduce the therapeutic effectiveness of corticosteroids in colitis treatment. This suggests that combining a TNF-alpha antagonist, *e.g*., the chimeric antibody REMICADE (infliximab), with a 15-PGDH inhibitor should be more effective in colitis (and IBD) treatment than using either agent alone.

In other embodiments, the 15-PGDH inhibitors and corticosteroids can be provided in a topical composition or formulation that is used to treat inflammation and/or aberrant immune system activity associated with medical conditions, such as atopic dermatitis, psoriasis, eczematous dermatitis, nummular dermatitis, irritant contact dermatitis, allergic contact dermatitis (such as poison ivy exposure, poison oak exposure, and poison sumac exposure), seborrheic dermatitis, stasis dermatitis, and other steroid responsive dermatoses.

In other embodiments, the 15-PGDH inhibitors and corticosteroids provided in a topical composition can be used to treat, for example, acne vulgaris, alopecia, alopecia greata, vitiligo, eczema, xerotic eczema, keratosis pilaris, lichen planus, lichen sclerosus, lichen striatus, lichen simplex chronicus, prurigo nodularis, discoid lupus erythematosus, lymphocytic infiltrate of Jessner/Kanof, lymphacytoma cutis, pyoderma gangrenosum, pruritis ani, sarcoidosis, chondrodermatitis nodularis helices, and other inflammatory dermatological disorders.

Medical conditions treated by the 15-PGDH inhibitors and corticosteroids can also include, for example, keloids, hypertrophic scars, pretibial myxedema and other infiltrative dermatological disorders. Additional medical conditions include, for example, granuloma annulare, necrobiosis lipoidica diabeticorum, sarcoidosis, and other noninfectious granulomas.

In other embodiments, the 15-PGDH inhibitor can be used as a glucocorticoid sensitizer to treat glucocorticoid insensitivity, restore corticosteroid sensitivity, enhance glucocorticoid sensitivity, and/or reverse the glucocorticoid insensitivity in a subject experiencing corticosteroid dependence or corticoid resistance or unresponsiveness or intolerance to corticosteroids. Therapeutic effects of the 15-PGDH inhibitors when used as a glucocorticoid sensitizer include any, but are not limited to, steroid-sparing in corticosteroid-dependent patients, better responsiveness or tolerance to corticosteroids, achieving efficacy by using a lower dose of corticosteroid, preventing individuals at risk for developing refractory responses or resistance or exacerbations in response to antigen exposures, infections, exercise, or irritants, achieving optimal immune functions, easier responses for the subject or patient when steroid administration is tapered or withdrawn, or after prolonged administration of corticosteroids, decreased risks for developing corticosteroid-related adverse events such as opportunistic infections, bone loss, pathologic fracture, diabetes, cataract, and combinations thereof.

In some embodiments, the 15-PGDH inhibitor can be administered to a subject in combination with the corticosteroid to treat glucocorticoid insensitivity, restore corticosteroid sensitivity, enhance glucocorticoid sensitivity, and/or reverse the glucocorticoid insensitivity in a subject experiencing corticosteroid dependence or corticoid resistance or unresponsiveness or intolerance to corticosteroids. The glucocorticoid insensitivity related conditions can include a range of immune-inflammatory disorders/diseases treated with steroids when the therapy fails to achieve disease control or is not effective or intolerant or dependent to corticosteroids, and combinations thereof.

In other embodiments, the 15-PGDH inhibitor and corticosteroid can be administered to a subject that exhibits one or more glucocorticoid insensitivity related diseases, disorders, or conditions selected from the group consisting of glucocorticoid resistant asthma, refractory rheumatoid arthritis, refractory inflammatory bowel disease, chronic obstructive pulmonary disease, acute respiratory distress syndrome, interstitial pulmonary fibrosis, cystic fibrosis, refractory ulcerative colitis, children with severe Crohn's disease, corticosteroid refractory asthma, desquamative interstitial pneumonia refractory to corticosteroid, refractory inflammatory myopathies, refractory myasthenia gravis, refractory pemphigus vulgaris, methotrexate-refractory RA patients, refractory nephrotic syndrome, refractory multiple sclerosis, refractory sprue-like disease, steroid-resistant sarcoidosis, refractory mucosal lesions of pemphigus vulgaris, refractory Schnitzler syndrome, resistant dermatitis of the head and neck, severe refractory atopic dermatitis, refractory Idiopathic thrombocytopenia purpura, refractory orbital myositis, refractory or recurrent lymphomas, critically ill patients with sepsis or acute respiratory distress syndrome (ARDS) and relative adrenal insufficiency, rosacea, polymyalgia rheumatic, giant cell arteritis, polymyositis, dermatomyositis, Kawasaki syndrome, Guillain-Barre syndrome, chronic inflammatory demyelinating polyneuropathy, multifocal motor neuropathy, Stiff man syndrome, corticosteroid dependent systemic lupus erythematosus, corticosteroid dependent multiple sclerosis, symptomatic corticosteroid dependent asthma, primary Sjogren's syndrome, systemic vasculitis, polymyositis, organ transplants, graft-versus-host disease, inflammatory diseases, autoimmune diseases, hyperproliferative diseases, lupus, osteoarthritis, rhinosinusitis, polyarteritis nodosa, Wegener's granulomatosis, giant cell arteritis, allergic rhinitis, urticaria, hereditary angioedema, tendonitis, bursitis, autoimmune chronic active hepatitis, cirrhosis, transplant rejection, psoriasis, dermatitus, malignancies, leukemia, myelomas, lymphomas, acute adrenal insufficiency, rheumatic fever, granulomatous disease, immune proliferation/apotosis, hypothalamic-pituitary-adrenal (HPA) axis suppression and regulation, hypercortisolemia, modulation of the Th1/Th2 cytokine balance, chronic kidney disease, spinal cord injury, cerebral edema, thrombocytopenia, Little's syndrome, Addison's disease, autoimmune hemolytic anemia, uveitis, pemphigus vulgaris, nasal polyps, sepsis, bacterial infections, viral infections, rickettsial infections, parasitic infections, type II diabetes, obesity, metabolic syndrome, depression, schizophrenia, mood disorders, Cushing's syndrome, anxiety, sleep disorders, memory and learning enhancement, glucocorticoid-induced glaucoma, atopic dermatitis, drug hypersensitivity reactions, serum sickness, bullous dermatitis herpetiformis, contact dermatitis, exfoliative erythroderma, mycosis fungoides, pemphigus, nonsuppurative thyroiditis, sympathetic ophthalmia, uveitis, ocular inflammatory conditions unresponsive to topical steroids, allergic bronchopulmonary aspergillosis, fulminating or disseminated pulmonary tuberculosis when used concurrently with appropriate chemotherapy, hypersensitivity pneumonitis, idiopathic bronchiolitis obliterans with organizing pneumonia, idiopathic eosinophilic pneumonias, idiopathic pulmonary fibrosis, pneumocystis carinii pneumonia (PCP) associated with hypoxemia occurring in an HIV(+) individual who is also under treatment with appropriate anti-PCP antibiotics, a diuresis or remission of proteinuria in nephrotic syndrome, without uremia, of the idiopathic type or that due to lupus erythematosus, ankylosing spondylitis, polymyalgia rheumatic, psoriatic arthritis, relapsing polychondritis, trichinosis with neurologic or myocardial involvement, and tuberculous meningitis.

The 15-PGDH inhibitors used in the methods described herein can be identified using assays in which putative inhibitor compounds are applied to cells expressing 15-PGDH and then the functional effects on 15-PGDH activity are determined. Samples or assays comprising 15-PGDH that are treated with a potential inhibitor are compared to control samples without the inhibitor to examine the extent of effect. Control samples (untreated with modulators) are assigned a relative 15-PGDH activity value of 100%. Inhibition of 15-PGDH is achieved when the 15-PGDH activity value relative to the control is about 80%, optionally 50% or 25%, 10%, 5% or 1%.

Agents tested as inhibitors of 15-PGDH can be any small chemical molecule or compound. Typically, test compounds will be small chemical molecules, natural products, or peptides. The assays are designed to screen large chemical libraries by automating the assay steps and providing compounds from any convenient source to assays, which are typically run in parallel (*e.g.,* in microtiter formats on microtiter plates in robotic assays).

In some embodiments, a15-PGDH inhibitor can include a compound having the following formula (VIII): 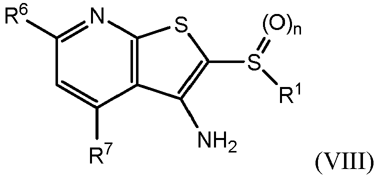
wherein n is 0-2;
R¹, R⁶, and R⁷ are the same or different and are each selected from the group consisting of hydrogen, substituted or unsubstituted C₁-C₂₄ alkyl, C₂-C₂₄ alkenyl, C₂-C₂₄ alkynyl, C₃-C₂₀ aryl, heteroaryl, heterocycloalkenyl containing from 5-6 ring atoms (wherein from 1-3 of the ring atoms is independently selected from N, NH, N(C₁-C₆ alkyl), NC(O) (C₁-C₆ alkyl), O, and S), C₆-C₂₄ alkaryl, C₆-C₂₄ aralkyl, halo, -Si(C₁-C₃ alkyl)₃, hydroxyl, sulfhydryl, C₁-C₂₄ alkoxy, C₂-C₂₄ alkenyloxy, C₂-C₂₄ alkynyloxy, C₃-C₂₀ aryloxy, acyl (including C₂-C₂₄ alkylcarbonyl (--CO-alkyl) and C₆-C₂₀ arylcarbonyl (-CO-aryl)), acyloxy (-O-acyl), C₂-C₂₄ alkoxycarbonyl (-(CO)-O-alkyl), C₆-C₂₀ aryloxycarbonyl (-(CO)-O-aryl), C₂-C₂₄ alkylcarbonato (-O-(CO)-O-alkyl), C₆-C₂₀ arylcarbonato (-O-(CO)-O-aryl), carboxy (-COOH), carboxylato (-COO⁻), carbamoyl (-(CO)-NH₂), C₁-C₂₄ alkyl-carbamoyl (-(CO)-NH(C₁-C₂₄ alkyl)), arylcarbamoyl (-(CO)-NH-aryl), thiocarbamoyl (-(CS)-NH₂), carbamido (-NH-(CO)-NH₂), cyano(-CN), isocyano (-N⁺C⁻), cyanato (-O-CN), isocyanato (-O-N⁺=C⁻), isothiocyanato (-S-CN), azido (-N=N⁺=N⁻), formyl (--(CO)--H), thioformyl (-(CS)--H), amino (--NH₂), C₁-C₂₄ alkyl amino, C₃-C₂₀ aryl amino, C₂-C₂₄ alkylamido (-NH-(CO)-alkyl), C₆-C₂₀ arylamido (-NH-(CO)-aryl), imino (-CR=NH where R is hydrogen, C₁-C₂₄ alkyl, C₅-C₂₀ aryl, C₆-C₂₄ alkaryl, C₆-C₂₄ aralkyl, etc.), alkylimino (-CR=N(alkyl), where R=hydrogen, alkyl, aryl, alkaryl, aralkyl, etc.), arylimino (-CR=N(aryl), where R=hydrogen, alkyl, aryl, alkaryl, etc.), nitro (-NO₂), nitroso (-NO), sulfo (-SO₂-OH), sulfonato (-SO₂-O⁻), C₁-C₂₄ alkylsulfanyl (-S-alkyl; also termed "alkylthio"), arylsulfanyl (-S-aryl; also termed "arylthio"), C₁-C₂₄ alkylsulfinyl (-(SO)-alkyl), C₃-C₂₀ arylsulfinyl (-(SO)-aryl), C₁-C₂₄ alkylsulfonyl (-SO₂-alkyl), C₃-C₂₀ arylsulfonyl (-SO₂-aryl), sulfonamide (-SO₂-NH2, - SO₂NY₂ (wherein Y is independently H, aryl or alkyl), phosphono (-P(O)(OH)₂), phosphonato (-P(O)(O⁻)₂), phosphinato (-P(O)(O⁻)), phospho (-PO₂), phosphino (--PH₂), polyalkylethers, phosphates, phosphate esters, groups incorporating amino acids or other moieties expected to bear positive or negative charge at physiological pH, combinations thereof, and wherein R⁶ and R⁷ may be linked to form a cyclic or polycyclic ring, wherein the ring is a substituted or unsubstituted aryl, a substituted or unsubstituted heteroaryl, a substituted or unsubstituted cycloalkyl, and a substituted or unsubstituted heterocyclyl; and pharmaceutically acceptable salts thereof.

15-PGDH inhibitors having formula (VIII) can be synthesized as shown:

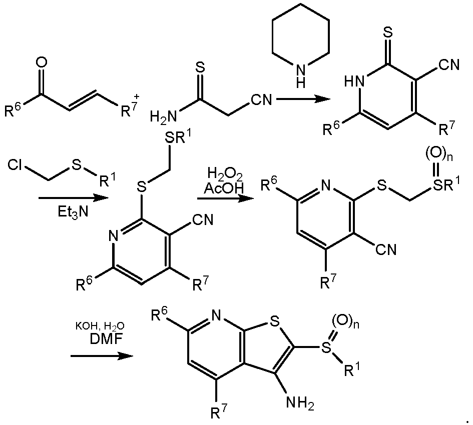

Any reaction solvent can be used in the above preparation process as long as it is not involved in the reaction. For example, the reaction solvent includes ethers such as diethyl ether, tetrahydrofuran and dioxane; halogenized hydrocarbons, such as dichloromethane and chloroform; amines such as pyridine, piperidine and triethylamine; alkylketones, such as acetone, methylethylketone and methylisobutyl; alcohols, such as methanol, ethanol and propanol; non-protonic polar solvent, such as N,N-dimethylformamide, N,N-dimethylacetamide, acetonitrile, dimethylsulfoxide and hexamethyl phosphoric acid triamide. Among non-reactive organic solvents that are ordinarily used in the organic synthesis, preferable solvents are those from which water generated in the reaction can be removed by a Dean-Stark trap. The examples of such solvents include, but are not limited to benzene, toluene, xylene and the like. The reaction product thus obtained may be isolated and purified by condensation, extraction and the like, which is ordinarily conducted in the field of the organic synthesis, if desired, by silica gel column chromatography. The individual enantiomers of PGDH inhibitors having the formula III can be separated by a preparative HPLC using chromatography columns containing chiral stationary phases.

Further, embodiments of this application include any modifications for the preparation method of the 15-PGDH inhibitors described above. In this connection, any intermediate product obtainable from any step of the preparation method can be used as a starting material in the other steps. Such starting material can be formed in situ under certain reaction conditions. Reaction reagents can also be used in the form of their salts or optical isomers.

Depending on the kinds of the substituents to be used in the preparation of the 15-PGDH inhibitors, and the intermediate product and the preparation method selected, novel 15-PGDH inhibitors can be in the form of any possible isomers such as substantially pure geometrical (cis or trans) isomers, optical isomers (enantiomers) and racemates.

The 15-PGDH inhibitor having formula (VIII) includes a compound with the following formula (IX): 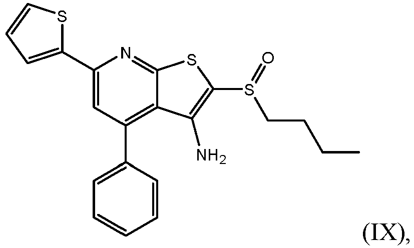 and pharmaceutically acceptable salts thereof.

Advantageously, the 15-PDGH inhibitor having formula (IX) was found to: i) inhibit recombinant 15-PGDH at 1 nM concentration; ii) inhibit 15-PGDH in cell lines at 100 nM concentration, iii) increase PGE₂ production by cell lines; iv) is chemically stable in aqueous solutions over broad pH range; v) is chemically stable when incubated with hepatocyte extracts, vi) is chemically stable when incubated with hepatocyte cell lines; vii) shows 253 minutes plasma half-life when injected IP into mice; and viii) shows no immediate toxicity over 24 hours when injected IP into mice at 0.6 µmole/per mouse and at 1.2 µmole/per mouse and also no toxicity when injected IP into mice at 0.3 µmole/per mouse twice daily for 21 days.

In other embodiments, the 15-PGDH inhibitor having formula (IX) can include a compound with the following formula (IXa): 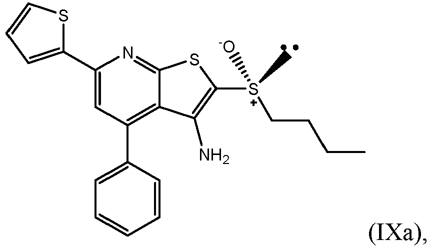 and pharmaceutically acceptable salts thereof.

In still other embodiments, the 15-PGDH inhibitor having formula (IX) can include a compound with the following formula (IXb): 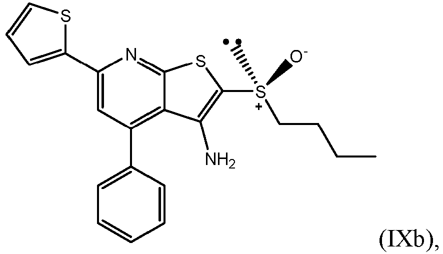 and pharmaceutically acceptable salts thereof.

In other embodiments, the 15-PDHG inhibitor can comprise a (+) or (-) optical isomer of a 15-PGDH inhibitor having formula (IX). In still other embodiments, the 15-PDHG inhibitor can comprise a mixture at least one of a (+) or (-) optical isomer of a 15-PGDH inhibitor having formula (IX). For example, the 15-PGDH inhibitor can comprise a mixture of: less than about 50% by weight of the (-) optical isomer of a 15-PGDH inhibitor having formula (IX) and greater than about 50% by weight of the (+) optical isomer of a 15-PGDH inhibitor having formula (IX), less than about 25% by weight of the (-) optical isomer of a 15-PGDH inhibitor having formula (IX) and greater than about 75% by weight of the (+) optical isomer of a 15-PGDH inhibitor having formula (IX), less than about 10% by weight of the (-) optical isomer of a 15-PGDH inhibitor having formula (IX) and greater than about 90% by weight of the (+) optical isomer of a 15-PGDH inhibitor having formula (IX), less than about 1% by weight of the (-) optical isomer of a 15-PGDH inhibitor having formula (IX) and greater than about 99% by weight of the (+) optical isomer of a 15-PGDH inhibitor having formula (IX), greater than about 50% by weight of the (-) optical isomer of a 15-PGDH inhibitor having formula (IX) and less than about 50% by weight of the (+) optical isomer of a 15-PGDH inhibitor having formula (IX), greater than about 75% by weight of the (-) optical isomer of a 15-PGDH inhibitor having formula (IX) and less than about 25% by weight of the (+) optical isomer of a 15-PGDH inhibitor having formula (IX), greater than about 90% by weight of the (-) optical isomer of a 15-PGDH inhibitor having formula (IX) and less than about 10% by weight of the (+) optical isomer of a 15-PGDH inhibitor having formula (IX), or greater than about 99% by weight of the (-) optical isomer of a 15-PGDH inhibitor having formula (IX) and less than about 1% by weight of the (+) optical isomer of a 15-PGDH inhibitor having formula (IX).

In a still further embodiment, the 15-PDGH inhibitor can consist essentially of or consist of the (+) optical isomer of a 15-PGDH inhibitor having formula (IX). In yet another embodiment, the PDGH inhibitor can consist essentially of or consist of the (-) optical isomer of a 15-PGDH inhibitor having formula (IX).

It will be appreciated that the other 15-PGDH inhibitors can be used in the methods described herein. These other 15-PGDH inhibitors can include known 15-PGDH inhibitors including, for example, tetrazole compounds of formulas (I) and (II), 2-alkylideneaminooxyacetamidecompounds of formula (I), heterocyclic compounds of formulas (VI) and (VII), and pyrazole compounds of formula (III) described in U.S. Patent Application Publication No. 2006/0034786 and U.S. Patent No. 7,705,041; benzylidene-1,3-thiazolidine compounds of formula (I) described in U.S. Patent Application Publication No. 2007/0071699; phenylfurylmethylthiazolidine-2,4-dione and phenylthienylmethylthiazolidine-2,4-dione compounds described in U.S. Patent Application Publication No. 2007/0078175; thiazolidenedione derivatives described in U.S. Patent Application Publication No. 2011/0269954; phenylfuran, phenylthiophene, or phenylpyrrazole compounds described in U.S. Patent No. 7,294,641, 5-(3,5-disubstituted phenylazo)-2-hydroxybenzene-acetic acids and salts and lactones described in U.S. Patent No. 4,725,676, and azo compounds described in U.S. Patent No. 4,889,846.

In certain embodiments, the corticosteroid is selected from the group consisting of aclovate, alclometasone dipropionate, amcinafel, amcinafide, amcinonide, aristocort A, augmented betamethasone dipropionate, beclamethasone, beclopmethasone dipropionate, betamethasone, betamethasone benzoate, betamethasone-17-benzoate, betamethasone dipropionate, betamethasone sodium phosphate and acetate, betamethasone valerate, betamethasone-17-valerate, chloroprednisone, clobetasol propionate, clobetasone propionate, clocortelone, cordran, corticosterone, cortisol, cortisol acetate, cortisol cypionate, cortisol sodium phosphate, cortisol sodium succinate, cortisone, cortisone acetate, cortodoxone, cyclocort, deflazacort, defluprednate, descinolone, desonide, desowen, desoximetasone, desoxycorticosterone acetate, desoxycorticosterone pivalate, 11-desoxycortisol, dexamethasone, dexamethasone acetate, dexamethasone sodium phosphate, dichlorisone, diflorasone diacetate, dihydroxycortisone, diprolen, diprolene, diprosone, esters of betamethasone, florone, flucetonide, flucloronide, flucortolone, fludrocortisone, fludrocortisone acetate, flumethalone, flumethasone, flumethasone pivalate, flunisolide, fluocinolone acetonide, fluocinolone acetonide acetate, fluocinonide, fluorametholone, fluorocortisone, fluperolone, fluprednisolone, flurandrenolide, fluroandrenolone acetonide, fluticasone propionate, fuprednisolone, halcinonide, halobetasol propionate, halog, hydrocortamate, hydrocortisone, hydrocortisone acetate, hydrocortisone butyrate, hydrocortisone valerate, hydrocortisone-17-valerate, kenalog, lidex, locold, locorten, maxiflor, medrysone, meprednisone, methylprednisolone, 6 α-methylprednisolone, methylprednisolone acetate, methylprednisolone sodium succinate, methylprednisone, mometasone furoate, paramethasone, paramethasone acetate, prednidone, prednisone, prednisolone, prednisolone acetate, prednisolone sodium phosphate, prednisolone sodium succinate, prednisolone tebutate, prednisone, psorcon, synalar, temovate, tetrahydrocortisol, topicort, topicort LP, triamcinolone, triamcinolone acetonide, triamcinolone diacetate, triamcinolone hexacotonide, tridesilone, valisone, and westcort.

In other embodiments, the corticosteroids to be used in combination with the 15-PGDH inhibitors described herein are prednisolone, methylprednisolone, dexamethasone, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone famesylate, ciclesonide, deprodone propionate, fluticasone, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, hydrocortisone acetate, hydrocortisone sodium succinate, prednisolone sodium phosphate and hydrocortisone probutate.

The 15-PGDH inhibitors and corticosteroids described herein can be provided in a pharmaceutical composition. A pharmaceutical composition containing the 15-PGDH inhibitors and corticosteroids described herein as an active ingredient may be manufactured by mixing the derivative with a pharmaceutically acceptable carrier(s) or an excipient(s) or diluting the 15-PGDH inhibitors and corticosteroids and TNF inhibitors described herein with a diluent in accordance with conventional methods. The pharmaceutical composition may further contain fillers, anti-cohesives, lubricants, wetting agents, flavoring agents, emulsifying agents, preservatives and the like. The pharmaceutical composition may be formulated into a suitable formulation in accordance with the methods known to those skilled in the art so that it can provide an immediate, controlled or sustained release of the 15-PGDH inhibitors and/or corticosteroids described herein after being administered into a mammal.

In some embodiments, the pharmaceutical composition may be formulated into a parenteral or oral dosage form. The solid dosage form for oral administration may be manufactured by adding excipient, if necessary, together with binder, disintegrants, lubricants, coloring agents, and/or flavoring agents, to the 15-PGDH inhibitors and corticosteroids described herein and shaping the resulting mixture into the form of tablets, sugar-coated pills, granules, powder or capsules. The additives that can be added in the composition may be ordinary ones in the art. For example, examples of the excipient include lactose, sucrose, sodium chloride, glucose, starch, calcium carbonate, kaolin, microcrystalline cellulose, silicate and the like. Exemplary binders include water, ethanol, propanol, sweet syrup, sucrose solution, starch solution, gelatin solution, carboxymethylcellulose, hydroxypropyl cellulose, hydroxypropyl starch, methylcellulose, ethylcellulose, shellac, calcium phosphonate and polypyrrolidone. Examples of the disintegrant include dry starch, sodium arginate, agar powder, sodium bicarbonate, calcium carbonate, sodium lauryl sulfate, stearic monoglyceride and lactose. Further, purified talc, stearates, sodium borate, and polyethylene glycol may be used as a lubricant; and sucrose, bitter orange peel, citric acid, tartaric acid, may be used as a flavoring agent. In some embodiments, the pharmaceutical composition can be made into aerosol formulations (*e.g*., they can be nebulized) to be administered via inhalation.

The 15-PGDH inhibitors and corticosteroids described herein described herein may be combined with flavoring agents, buffers, stabilizing agents, and the like and incorporated into oral liquid dosage forms such as solutions, syrups or elixirs in accordance with conventional methods. One example of the buffers may be sodium citrate. Examples of the stabilizing agents include tragacanth, acacia and gelatin.

In some embodiments, the 15-PGDH inhibitors and corticosteroids described herein described herein may be incorporated into an injection dosage form, for example, for a subcutaneous, intramuscular or intravenous route by adding thereto pH adjusters, buffers, stabilizing agents, relaxants, topical anesthetics. Examples of the pH adjusters and the buffers include sodium citrate, sodium acetate and sodium phosphate. Examples of the stabilizing agents include sodium pyrosulfite, EDTA, thioglycolic acid and thiolactic acid. The topical anesthetics may be procaine HCl, lidocaine HCl and the like. The relaxants may be sodium chloride, glucose and the like.

In other embodiments, the 15-PGDH inhibitors and corticosteroids described herein described herein may be incorporated into suppositories in accordance with conventional methods by adding thereto pharmaceutically acceptable carriers that are known in the art, for example, polyethylene glycol, lanolin, cacao butter or fatty acid triglycerides, if necessary, together with surfactants such as Tween.

The pharmaceutical composition may be formulated into various dosage forms as discussed above and then administered through various routes including an oral, inhalational, transdermal, subcutaneous, intravenous or intramuscular route. The dosage can be a pharmaceutically or therapeutically effective amount.

Therapeutically effective dosage amounts of the 15-PGDH inhibitor and corticosteroids described herein may be present in varying amounts in various embodiments. For example, in some embodiments, a therapeutically effective amount of the 15-PGDH inhibitor may be an amount ranging from about 10-1000 mg (*e.g.,* about 20 mg-1,000 mg, 30 mg-1,000 mg, 40 mg-1,000 mg, 50 mg-1,000 mg, 60 mg-1,000 mg, 70 mg-1,000 mg, 80 mg-1,000 mg, 90 mg-1,000 mg, about 10-900 mg, 10-800 mg, 10-700 mg, 10-600 mg, 10-500 mg, 100-1000 mg, 100-900 mg, 100-800 mg, 100-700 mg, 100-600 mg, 100-500 mg, 100-400 mg, 100-300 mg, 200-1000 mg, 200-900 mg, 200-800 mg, 200-700 mg, 200-600 mg, 200-500 mg, 200-400 mg, 300-1000 mg, 300-900 mg, 300-800 mg, 300-700 mg, 300-600 mg, 300-500 mg, 400 mg-1,000 mg, 500 mg-1,000 mg, 100 mg-900 mg, 200 mg-800 mg, 300 mg-700 mg, 400 mg-700 mg, and 500 mg-600 mg). In some embodiments, the 15-PGDH inhibitor is present in an amount of or greater than about 10 mg, 50 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg. In some embodiments, the 15-PGDH inhibitor is present in an amount of or less than about 1000 mg, 950 mg, 900 mg, 850 mg, 800 mg, 750 mg, 700 mg, 650 mg, 600 mg, 550 mg, 500 mg, 450 mg, 400 mg, 350 mg, 300 mg, 250 mg, 200 mg, 150 mg, or 100 mg.

In other embodiments, a therapeutically effective amount of the corticosteroid may be an amount ranging from about 10-1000 mg (*e.g.,* about 20 mg-1,000 mg, 30 mg-1,000 mg, 40 mg-1,000 mg, 50 mg-1,000 mg, 60 mg-1,000 mg, 70 mg-1,000 mg, 80 mg-1,000 mg, 90 mg-1,000 mg, about 10-900 mg, 10-800 mg, 10-700 mg, 10-600 mg, 10-500 mg, 100-1000 mg, 100-900 mg, 100-800 mg, 100-700 mg, 100-600 mg, 100-500 mg, 100-400 mg, 100-300 mg, 200-1000 mg, 200-900 mg, 200-800 mg, 200-700 mg, 200-600 mg, 200-500 mg, 200-400 mg, 300-1000 mg, 300-900 mg, 300-800 mg, 300-700 mg, 300-600 mg, 300-500 mg, 400 mg-1,000 mg, 500 mg-1,000 mg, 100 mg-900 mg, 200 mg-800 mg, 300 mg-700 mg, 400 mg-700 mg, and 500 mg-600 mg). In some embodiments, the corticosteroid is present in an amount of or greater than about 10 mg, 50 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg. In some embodiments, the corticosteroid is present in an amount of or less than about 1000 mg, 950 mg, 900 mg, 850 mg, 800 mg, 750 mg, 700 mg, 650 mg, 600 mg, 550 mg, 500 mg, 450 mg, 400 mg, 350 mg, 300 mg, 250 mg, 200 mg, 150 mg, or 100 mg.

In other embodiments, a therapeutically effective dosage amount of the 15-PGHD inhibitor and/or the corticosteroid may be, for example, about 0.001 mg/kg weight to 500 mg/kg weight, *e.g*., from about 0.001 mg/kg weight to 400 mg/kg weight, from about 0.001 mg/kg weight to 300 mg/kg weight, from about 0.001 mg/kg weight to 200 mg/kg weight, from about 0.001 mg/kg weight to 100 mg/kg weight, from about 0.001 mg/kg weight to 90 mg/kg weight, from about 0.001 mg/kg weight to 80 mg/kg weight, from about 0.001 mg/kg weight to 70 mg/kg weight, from about 0.001 mg/kg weight to 60 mg/kg weight, from about 0.001 mg/kg weight to 50 mg/kg weight, from about 0.001 mg/kg weight to 40 mg/kg weight, from about 0.001 mg/kg weight to 30 mg/kg weight, from about 0.001 mg/kg weight to 25 mg/kg weight, from about 0.001 mg/kg weight to 20 mg/kg weight, from about 0.001 mg/kg weight to 15 mg/kg weight, from about 0.001 mg/kg weight to 10 mg/kg weight.

In still other embodiments, a therapeutically effective dosage amount of the 15-PGHD inhibitor and/or the corticosteroid may be, for example, about 0.0001 mg/kg weight to 0.1 mg/kg weight, e.g. from about 0.0001 mg/kg weight to 0.09 mg/kg weight, from about 0.0001 mg/kg weight to 0.08 mg/kg weight, from about 0.0001 mg/kg weight to 0.07 mg/kg weight, from about 0.0001 mg/kg weight to 0.06 mg/kg weight, from about 0.0001 mg/kg weight to 0.05 mg/kg weight, from about 0.0001 mg/kg weight to about 0.04 mg/kg weight, from about 0.0001 mg/kg weight to 0.03 mg/kg weight, from about 0.0001 mg/kg weight to 0.02 mg/kg weight, from about 0.0001 mg/kg weight to 0.019 mg/kg weight, from about 0.0001 mg/kg weight to 0.018 mg/kg weight, from about 0.0001 mg/kg weight to 0.017 mg/kg weight, from about 0.0001 mg/kg weight to 0.016 mg/kg weight, from about 0.0001 mg/kg weight to 0.015 mg/kg weight, from about 0.0001 mg/kg weight to 0.014 mg/kg weight, from about 0.0001 mg/kg weight to 0.013 mg/kg weight, from about 0.0001 mg/kg weight to 0.012 mg/kg weight, from about 0.0001 mg/kg weight to 0.011 mg/kg weight, from about 0.0001 mg/kg weight to 0.01 mg/kg weight, from about 0.0001 mg/kg weight to 0.009 mg/kg weight, from about 0.0001 mg/kg weight to 0.008 mg/kg weight, from about 0.0001 mg/kg weight to 0.007 mg/kg weight, from about 0.0001 mg/kg weight to 0.006 mg/kg weight, from about 0.0001 mg/kg weight to 0.005 mg/kg weight, from about 0.0001 mg/kg weight to 0.004 mg/kg weight, from about 0.0001 mg/kg weight to 0.003 mg/kg weight, from about 0.0001 mg/kg weight to 0.002 mg/kg weight. In some embodiments, the therapeutically effective dose may be 0.0001 mg/kg weight, 0.0002 mg/kg weight, 0.0003 mg/kg weight, 0.0004 mg/kg weight, 0.0005 mg/kg weight, 0.0006 mg/kg weight, 0.0007 mg/kg weight, 0.0008 mg/kg weight, 0.0009 mg/kg weight, 0.001 mg/kg weight, 0.002 mg/kg weight, 0.003 mg/kg weight, 0.004 mg/kg weight, 0.005 mg/kg weight, 0.006 mg/kg weight, 0.007 mg/kg weight, 0.008 mg/kg weight, 0.009 mg/kg weight, 0.01 mg/kg weight, 0.02 mg/kg weight, 0.03 mg/kg weight, 0.04 mg/kg weight, 0.05 mg/kg weight, 0.06 mg/kg weight, 0.07 mg/kg weight, 0.08 mg/kg weight, 0.09 mg/kg weight, or 0.1 mg/kg weight. The effective dose for a particular individual can be varied (*e.g*., increased or decreased) over time, depending on the needs of the individual.

In some embodiments, a therapeutically effective dosage of the 15-PGHD inhibitor and/or the corticosteroid may be a dosage of 10 µg/kg/day, 50 µg/kg/day, 100 µg/kg/day, 250 µg/kg/day, 500 µg/kg/day, 1000 µg/kg/day or more. In various embodiments, the amount of the 15-PGDH inhibitor and/or corticosteroid is sufficient to provide a dosage to a patient of between 0.01 µg/kg and 10 µg/kg; 0.1 µg/kg and 5 µg/kg; 0.1 µg/kg and 1000 µg/kg; 0.1 µg/kg and 900 µg/kg; 0.1 µg/kg and 900 µg/kg; 0.1 µg/kg and 800 µg/kg; 0.1 µg/kg and 700 µg/kg; 0.1 µg/kg and 600 µg/kg; 0.1 µg/kg and 500 µg/kg; or 0.1 µg/kg and 400 µg/kg.

Particular doses or amounts to be administered in accordance with the present invention may vary, for example, depending on the nature and/or extent of the desired outcome, on particulars of route and/or timing of administration, and/or on one or more characteristics (*e.g*., weight, age, personal history, genetic characteristic, lifestyle parameter, severity of cardiac defect and/or level of risk of cardiac defect, etc., or combinations thereof). Such doses or amounts can be determined by those of ordinary skill. In some embodiments, an appropriate dose or amount is determined in accordance with standard clinical techniques. For example, in some embodiments, an appropriate dose or amount is a dose or amount sufficient to reduce a disease severity index score by 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100% or more. For example, in some embodiments, an appropriate dose or amount is a dose or amount sufficient to reduce a disease severity index score by 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100%. Alternatively or additionally, in some embodiments, an appropriate dose or amount is determined through use of one or more *in vitro* or *in vivo* assays to help identify desirable or optimal dosage ranges or amounts to be administered.

Various embodiments may include differing dosing regimen. In some embodiments, the 15-PGDH inhibitor and corticosteroids described herein can be administered via continuous infusion. In some embodiments, the continuous infusion is intravenous. In other embodiments, the continuous infusion is subcutaneous. Alternatively or additionally, in some embodiments, the 15-PGDH inhibitor can be administered bimonthly, monthly, twice monthly, triweekly, biweekly, weekly, twice weekly, thrice weekly, daily, twice daily, or on another clinically desirable dosing schedule. The dosing regimen for a single subject need not be at a fixed interval, but can be varied over time, depending on the needs of the subject.

For topical application, the composition can be administered in the form of aqueous, alcoholic, aqueous-alcoholic or oily solutions or suspensions, or of a dispersion of the lotion or serum type, of emulsions that have a liquid or semi-liquid consistency or are pasty, obtained by dispersion of a fatty phase in an aqueous phase (O/W) or vice versa (W/O) or multiple emulsions, of a free or compacted powder to be used as it is or to be incorporated into a physiologically acceptable medium, or else of microcapsules or microparticles, or of vesicular dispersions of ionic and/or nonionic type. It may thus be in the form of a salve, a tincture, milks, a cream, an ointment, a powder, a patch, an impregnated pad, a solution, an emulsion or a vesicular dispersion, a lotion, aqueous or anhydrous gels, a spray, a suspension, a shampoo, an aerosol or a foam. It may be anhydrous or aqueous. It may also comprise solid preparations constituting soaps or cleansing cakes.

Pharmaceutical compositions including the 15-PGDH inhibitor and corticosteroids described herein can additionally contain, for example, at least one compound chosen from prostaglandins, in particular prostaglandin PGE₁, PGE₂, their salts, their esters, their analogues and their derivatives, in particular those described in WO 98/33497, WO 95/11003, JP 97-100091, JP 96-134242, in particular agonists of the prostaglandin receptors. It may in particular contain at least one compound such as the agonists (in acid form or in the form of a precursor, in particular in ester form) of the prostaglandin F₂α receptor, such as for example latanoprost, fluprostenol, cloprostenol, bimatoprost, unoprostone, the agonists (and their precursors, in particular the esters such as travoprost) of the prostaglandin E₂ receptors such as 17-phenyl PGE₂, viprostol, butaprost, misoprostol, sulprostone, 16,16-dimethyl PGE₂, 11-deoxy PGE₁, 1-deoxy PGE₁, the agonists and their precursors, in particular esters, of the prostacycline (IP) receptor such as cicaprost, iloprost, isocarbacycline, beraprost, eprostenol, treprostinil, the agonists and their precursors, in particular the esters, of the prostaglandin D₂ receptor such as BW245C ((4S)-(3-[(3R,S)-3-cyclohexyl-3-isopropyl]-2,5-dioxo)-4-imidazolidinehept- anoic acid), BW246C ((4R)-(3-[(3R,S)-3-cyclohexyl-3-isopropyl]-2,5-dioxo)-4-imidazolidinehept- anoic acid), the agonists and their precursors, in particular the esters, of the receptor for the thromboxanes A2 (TP) such as I-BOP ([1S-[1a,2a(Z), 3b(1E,3S),4a]]-7-[3-[3-hydroxy-4-[4-(iodophenoxy)-1-butenyl]-7-oxabicyclo- [2.2.1]hept-2-yl]-5-heptenoic acid).

Advantageously, the composition can include at least one 15-PGDH inhibitor and corticosteroid as defined above and at least one prostaglandin or one prostaglandin derivative such as for example the prostaglandins of series 2 including in particular PGF_{2α} and PGE₂ in saline form or in the form of precursors, in particular of the esters (example isopropyl esters), their derivatives such as 16,16-dimethyl PGE₂, 17-phenyl PGE₂ and 16,16-dimethyl PGF_{2α} 17-phenyl PGF_{2α}, prostaglandins of series 1 such as 11-deoxyprostaglandin E1, 1-deoxyprostaglandin E1 in saline or ester form, is their analogues, in particular latanoprost, travoprost, fluprostenol, unoprostone, bimatoprost, cloprostenol, viprostol, butaprost, misoprostol, their salts or their esters.

The invention is further illustrated by the following examples, which is not intended to limit the scope of the claims.

### Example 1

### Analysis of effect of SW033291 on dextan sodium sulfate (DSS) induced colitis

This Example provides data from studies of the effect of SW033291 on prevention of induction of colitis in the dextran sodium sulfate (DSS) treated mouse. In the study, 8-12 week old FVB male mice were fed with 2% DSS in drinking water for days 1-7, and then switched to normal drinking water beginning on day 8, and continued through day 22. Mice were treated with twice daily SW033291 5 mg/kg IP in a vehicle of 10% Ethanol, 5% Cremophor EL, 85% D5W, at 125 µg/200ul, versus with vehicle alone. Clinical scoring (body weight, rectal bleeding, stool consistency) was recorded daily, endoscopic scoring (ulcer number, mucosal thickening, and vascular pattern) was assessed on days 8, 11, 15. Mice were sacrificed on days 1, 8, 15 and 22 for assessment of colon length, colon weight, ulcer number, ulcer area, and crypt damage.

Table 1 shows a summary of the baseline properties of age and weight of the 24 SW033291 treated mice and the 24 control group mice used in the study. Also provided are baseline characteristics of 4 FVB male 15-PGDH knockout (KO) mice that are used as a comparator group.

**TABLE 1**

| FVB PGDH WT/ KO male mice | | | | |
|---|---|---|---|---|
| 8-12 weeks old | | | | |
| DSS Study | WT-Control | WT-Treatment | KO | p-value |
| Number | 24 | 24 | 4 | |
| Sex | M | M | M | |
| Age (Days) | 74.1+3.7 | 74.2+4.0 | 73.9±3.4 | 0.655 |
| Weight (gm) | 26.3±1.19 | 26.8±1.78 | 27.4±1.4 | 0.391 |

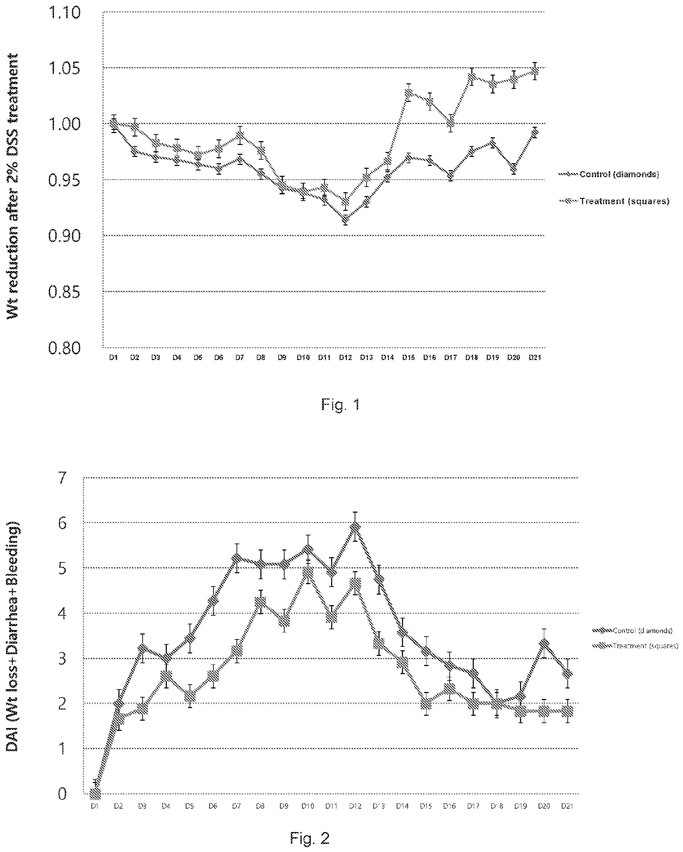

Fig. 1 shows a graph of the average changes from baseline weight of the cohort of control versus SW033291 treated mice across the 22 days of the study. SW033291 treated mice (squares) show greater weight at all time points, and in particular, show faster weight gain after washout of DSS then do the control mice (diamonds), P=0.001.

Fig. 2 shows a graph of the daily Disease Activity Index (DAI) of the cohort of control (diamonds) versus SW033291 treated mice across (squares) the 22 days of the study. The Disease Activity Index is calculated as an equally weighted average of the change from baseline weight, the consistency of stool, and the presence of rectal bleeding, with each component normalized to span an identical numerical range. SW033291 treated mice (squares) show a lower Disease Activity Index than do control (diamonds) on each day of the study, P<0.001.

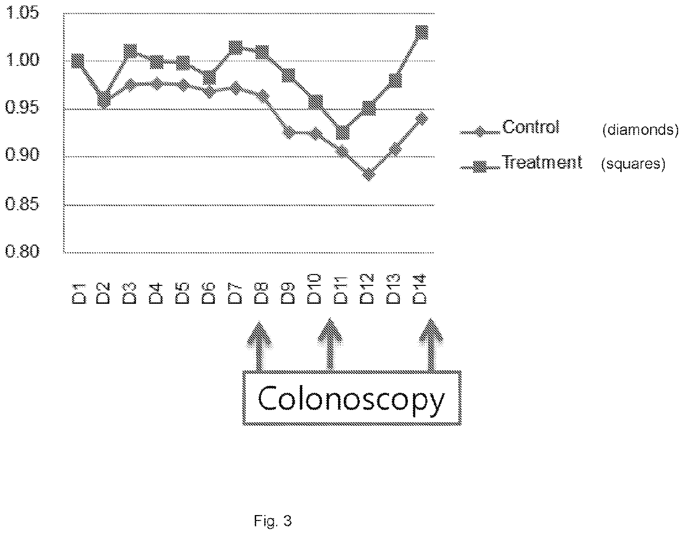

Fig. 3 shows the design of the study in which colonoscopic examination of the left colon, up to the splenic flexure, was performed on live mice on days 8, 11 and 15, under isoflurane anesthesia. Daily weights of these SW033291 treated (squares) and untreated mice (diamonds) were also recorded and are shown. In addition, post-mortem colonoscopy of the full colon was performed on two SW033291 treated (squares) and two control treated mice (diamonds) on day 15, with findings confirming that DSS induced ulcerations are largely confined to the descending colon distal to the splenic flexure.

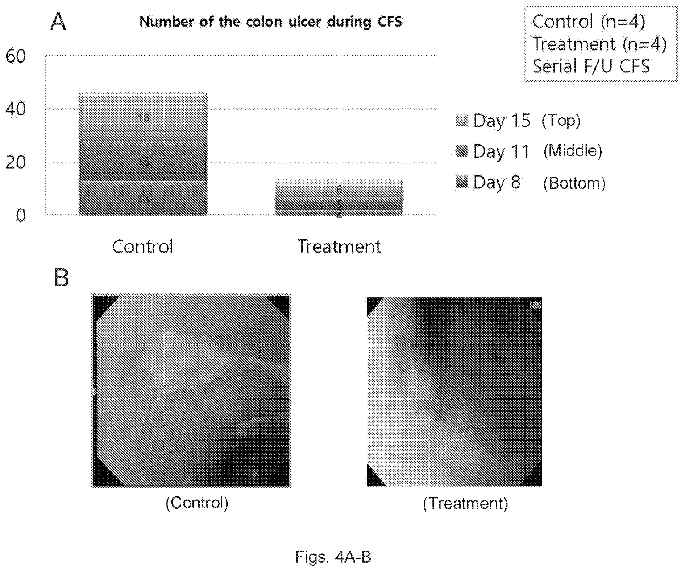

Figs. 4(A-B) show at bottom left the colon as visualized during colonoscopy of a DSS treated control mouse that shows loss of the mucosal vascular pattern and a gross ulceration (Fig. 4B). At bottom right is shown the colonoscopic findings of a DSS treated mouse receiving SW033291, with only a small ulcer and with maintenance of the normal mucosal vascular pattern otherwise (Fig. 4B). Fig. 4A is a graph showing numbers of ulcers present on days 8 (bottom), 11 (middle), and 15 (top) in the control versus SW033291 treated mice. SW033291 treatment prevents two-thirds of ulcer formation. Additional studies of 15-PGDH knockout mice show that 15-PGDH gene knockout prevents 95% of colon ulcer formation. These findings support that the colitis prevention activity of SW033291 is mediated through its activity as a 15-PGDH inhibitor, and suggest further modifications of drug dosing and delivery may provide added colitis prevention and would also be expected to protect from other forms of intestinal injury that would include toxicity from radiation, toxicity from chemotherapy, and chemotherapy induced mucositis.

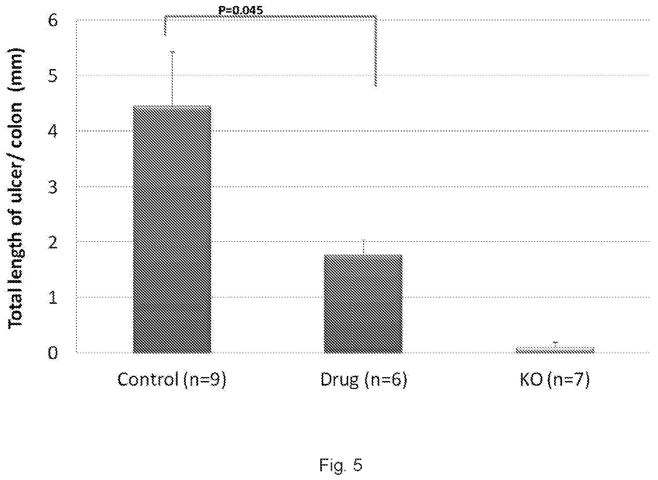

Fig. 5 shows quantitation of ulcer burden on day 15 of DSS treated mice as determined by embedding the full length of the formalin fixed colons of mice in paraffin blocks, and then microscopic inspection of a random 5 µm section along the full colon length for visualization and measurement of ulcerated mucosa. The graph shows that the average length of ulcerated mucosa is 4.48mm per colon section in control mice (N=9 mice) and is reduced by 61% to a length of 1.74mm per colon section in SW033291 (drug) treated mice (N=6 mice), P=0.045. Again, 15-PGDH gene knockout (KO) is highly effective in preventing colon ulceration, supporting that the therapeutic effect of SW033291 is mediated through inhibition of 15-PGDH.

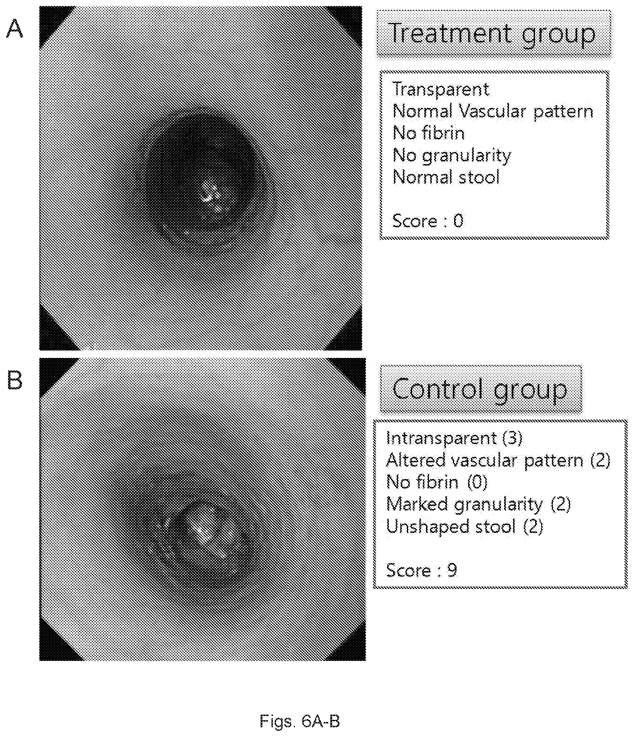

Figs. 6(A-B) show examples of scoring murine colonic mucosa according to the Murine Endoscopic Index of Colitis Severity (MEICS) (Becker C. et al. Gut 2005; 54: 950-954). Fig. 6A shows the colonoscopic findings and MEICS scoring for a DSS treated mouse receiving SW033291. Fig. 6B the colonoscopic findings and MEICS scoring of a DSS treated mouse receiving vehicle only.

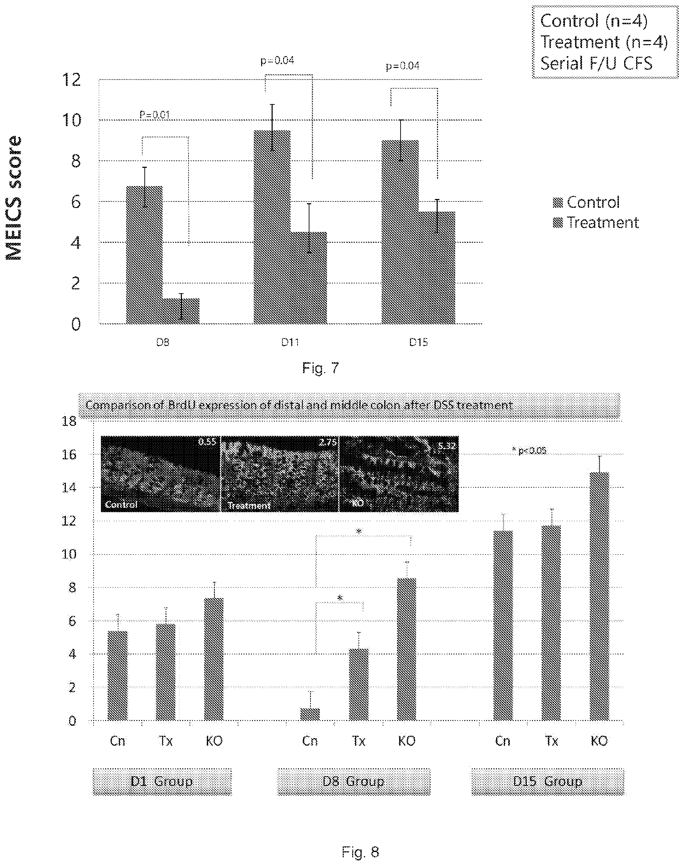

Fig. 7 shows graphs of the MEICS scores for DSS treated mice receiving SW033291 (treatment, right) versus vehicle (control, left). MEICS scores show significantly less colitis activity in SW033291 treated mice on days 8, 11 and 15 of the study.

In addition to the gross visual inspection and scoring of colitis activity by the MEICS index, full length colons of mice were formalin fixed and paraffin embedded, and microscopic scoring of crypt damage was performed using the 0 - 4 severity scale of Cooper HS. Et al., Lab Invest. 1993;69:238-249. For this analysis, the colons were divided into 3 segments of proximal, middle, and distal colon, each approximately 1.6cm in length, with each segment was further subdivided into 4 sections each approximately 4mm in length. For each section the crypt damage severity score was multiplied by the length in mm of the damaged area, creating a 0-16 cryptitis severity index. An average cryptitis severity index was calculated for each segment (proximal, middle, and distal colon), and the summed whole colon cryptitis severity index was determined on a scale of 0-48 for each mouse colon. In parallel with the visual MEICS score, the microscopic cryptitis severity index on day 8 of the DSS protocol was significantly greater in control mice (value of 9.49) than in the SW033291 treated mice (value of 3.16), P<0.05 (data described but not shown in the figure).

Fig. 8 shows assessment of the effect of SW033291 on maintaining DNA synthesis in the colonic mucosa of DSS treated mice. Mice were injected with BrdU at 100mg/kg IP 3 hours before sacrifice and then full length colons were formalin fixed and embedded in paraffin. S-phase cells, that have incorporated BrdU into DNA, were visualized by immuno-fluorescent staining of 5 µm thick sections with an antibody that detects the BrdU. Colonic crypts were visualized by immuno-fluorescent staining with an antibody to the epithelial marker E-Cadherin. Photographic insets show photomicrographs of high powered fields taken from the mid-colon on day 8 of the DSS protocol from control mice, SW033291 treated mice (treatment) and 15-PGDH knockout mice (KO). Red immune-fluorescence identifies BrdU positive nuclei, and green immune-fluorescence identifies E-Cadherin positive colonocytes. The number of BrdU positive cell per crypt is determined by counting the number of dual labeled red and green cells per average crypt. Green only cells that are not in S-phase are not counted, and red only cells, that are likely stromal cells outside of crypts, are also not counted. On the photomicrograph shown crypts are displayed as vertically oriented in control and SW033291 treated mice, and crypts are displayed as horizontally oriented in the 15-PGDH knockout mice. In the photographs the numbers of S-phase cells are fewest in the control mice and are increased in the SW033291 treated mice, and increased further in the knockout mice. In the particular photographs shown, the crypts from control mice both lack S-phase cells and are also visually decreased in height; whereas, crypt height is increased in the crypts shown from SW033291 treated mice, and crypt heights is increased further in the crypts shown from 15-PGDH knockout mice. The graph depicts the sum of the average number of BrdU positive cells per crypt in the distal colon plus the average number of BrdU positive cells per crypt middle colons of control (Cn), SW033219 treated (Tx) , and 15-PGDH knockout mice (KO) on day 1, day 8 , and day 15 of the DSS treatment protocol. On day 8, SW033291 treated mice demonstrate 5.7-fold greater numbers of BrdU positive cells than do control mice, which have lost 85% of the day 1 value of BrdU positive cells per crypt. 15-PGDH knockout mice show no loss of BrdU positive cells in the crypt on day 8, consistent with the protective effect of SW033291 being mediated by inhibition of 15-PGDH.

Table 2 shows a summary of colon length (in cm) in DSS treated mice sacrificed on days 8, 15 and 22, in SW033291 treated mice, versus vehicle treated control mice, versus 15-PGDH knockout (KO) mice, where shortening of the colon is a measure of disease activity.

**TABLE 2**

| Colon length shortening may be correlated to severity of the colon ulceration | | | | |
|---|---|---|---|---|
| Time Point | Control | SW033291 | KO | P-value |
| Baseline | 8.3+0.2 | 8.4+0.2 | | 0.71 |
| Day 8 | 6.6+0.4 | 6.6+0.1 | | 1.0 |
| Day 15 | 7.1+0.1 | 7.5+0.1 | 8.5+0.1 | 0.001 |
| Day 22 | 7.4+0.2 | 8.6+0.3 | | 0.012 |

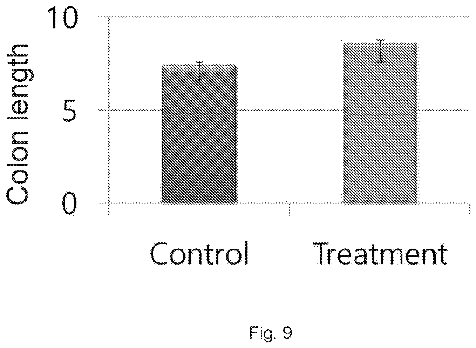

Vehicle treated control mice show significantly greater colon shortening at day 22 versus SW033291 treated mice, P=0.012. This comparison is also shown graphically in Fig. 9.

Table 3 shows a summary on day of sacrifice of mouse weights (gms) and colon lengths (cm) for DSS treated mice receiving SW033291 or vehicle control.

**TABLE 3**

| Wt @ sacrifice-gm Time Point | Vehicle | SW033291 | KO |
|---|---|---|---|
| Baseline | 26.3+0.7 | 25.9+0.7 | |
| Day 8 | 25.4+0.7 | 26.4+0.5 | |
| Day 15 | 24.4+0.5 | 25.2+0.9 | 29.2+1.3 |
| Day 22 * | 26.3+0.7 | 28.2+0.5 | |
| Colon length-cm Time Point | | | |
| Baseline | 8.3+0.2 | 8.4+0.2 | |
| Day 8 | 6.6+0.4 | 6.6+0.1 | |
| Day 15 | 7.1+0.1 | 7.5+0.1 | 8.5+0.1 |
| Day 22 * | 7.4+0.2 | 8.6+0.3 | |

On day 22 SW033291 treated mice show greater body weight and greater colon lengths, indicative of therapeutic effect of SW033291 in protecting against DSS induced colitis.

### Example 2

### Identifying Signaling Networks Associated with 15-PGDH Expression

In order to identify signaling networks that are significantly correlated with 15-PGDH gene expression in colon tissues, we first to comprehensively characterized global pathway network activities across 16 normal colon tissue samples using an integrative pathway network modeling framework, PARADIGM. (Vaske, C.J., et al. Inference of patient-specific pathway activities from multi-dimensional cancer genomics data using PARADIGM. Bioinformatics 26, i237-245 (2010).) The PARADIGM analytics framework leverages gene expression measurements for a given sample in order to explicitly model regulatory relationships detailed in a given signaling network and estimate the biological activity state of each of the signaling network components in the tissue sample. (Varadan, V., Mittal, P., Vaske, C.J. & Benz, S.C. The integration of biological pathway knowledge in cancer genomics: A review of existing computational approaches. IEEE Signal Processing Magazine 29, 35-50 (2012); Cancer Genome Atlas, N. Comprehensive molecular characterization of human colon and rectal cancer. Nature 487, 330-337 (2012); Cancer Genome Atlas, N. Comprehensive molecular portraits of human breast tumours. Nature 490, 61-70 (2012); tlas, T.C.G. Integrated genomic analyses of ovarian carcinoma. Nature 474, 609-615 (2011)). PARADIGM incorporates known signaling network information curated within public databases such as the National Cancer Institute's Pathway Interaction Database (NCI-PID), Reactome and BioCarta pathway databases, resulting in a merged signaling network structure (SuperPathway) containing over 17000 concepts representing 7324 proteins, 1574 protein families, 7813 complexes, and 586 processes. (Schaefer, C.F., et al. PID: the Pathway Interaction Database. Nucleic acids research 37, D674-679 (2009); Croft, D., et al. The Reactome pathway knowledgebase. Nucleic acids research 42, D472-477 (2014)). Thus, PARADIGM leverages gene expression data obtained for genes within the SuperPathway network to infer sample-specific activity levels, called Integrated Pathway Levels (IPLs) for each network component in the SuperPathway network. The IPLs are typically distributed between -1 and +1, with negative IPLs corresponding to lower activity and positive IPLs corresponding to higher pathway-specific activity.

Accordingly, we used PARADIGM to analyze normalized, log2-transformed gene expression values across normal colon tissue samples (*N* = 16) resulting in the estimation of Integrated Pathway Levels (IPL) for each component of the SuperPathway network, and then evaluated the correlation of the IPLs across all components in the SuperPathway with the normalized 15-PGDH gene expression. The extent and statistical significance of the correlation was determined using the Spearman's *rho* statistic. Pathway network components with a Spearman correlation p-value ≤ 0.01 were considered significant and the resulting sub-networks along with their regulatory relationships were plotted using Cytoscape. (Shannon, P., et al. Cytoscape: a software environment for integrated models of biomolecular interaction networks. Genome research 13, 2498-2504 (2003)). The resulting interconnected component sub-networks provide insights into transcription factor activities associated with 15-PGDH gene expression across normal colon tissues.

In order to further evaluate the likelihood of identifying a sub-network of a given size purely by chance, we performed 10,000 randomization experiments. In each iteration, we randomly selected the same number of network components from the SuperPathway as originally identified to be significantly associated with 15-PGDH expression. Subsequently, for each iteration, we determined the sizes and numbers of connected sub-networks derived from these random component selections. The resulting distribution of sub-network sizes obtained from the 10,000 random iterations were modeled as a Poisson distribution, thus allowing us to estimate the probability of obtaining a sub-network of a given size purely by chance.

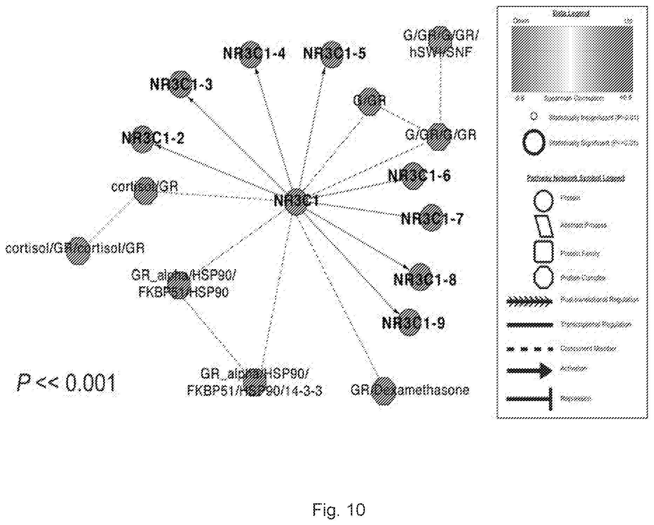

Fig. 10 illustrates identifying that Glucocorticoid receptor NR3C1-centered sub-network activities are significantly correlated with 15-PGDH gene expression in normal human colons via examining PARADIGM SuperPathway sub-networks whose activities are significantly correlated with 15-PGDH gene expression in normal colon tissues. Pathway components showing significant activity correlation with 15-PGDH gene expression across 16 normal colon cancer tissues (Spearman Correlation P-Value ≤ 0.01) are plotted along with their regulatory relationships in shades of red (positively correlated) and green (negatively correlated), with darker colors corresponding to higher absolute correlation. The size of the node corresponds to the statistical significance of the correlation. The p-value assigned to each sub-network is the probability of obtaining a sub-network of this size purely by chance.

### Example 3

### Combination of a Corticosteroid and an Inhibitor of 15-PGDH

We have previously demonstrated that SW033291, an inhibitor of 15-PGDH, has activity in treatment of DSS induced colitis, a murine model of ulcerative colitis. This example provides new findings showing inhibitors of 15-PGDH synergistically enhance corticosteroid treatment of DSS induced colitis, a murine model of ulcerative colitis.

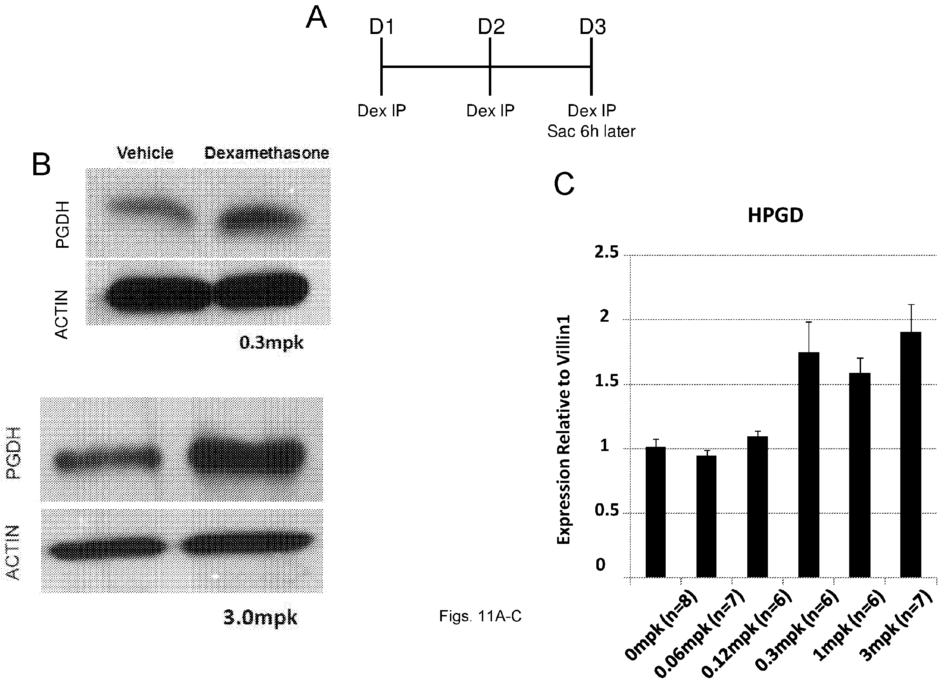

Fig. 11A shows a schema of the study in which mice received three daily doses of dexamethasone and were sacrificed 6 hours after the third dose for analysis. Fig. 11B shows representative western blot analysis showing dexamethasone induction of 15-PGDH protein in mouse colon, at two different doses of dexamethasone. Fig. 11C is a graphical summary of real time RT-PCR from all mice in the study, showing an approximate doubling of colon 15-PGDH expression level by dexamethasone treatment.

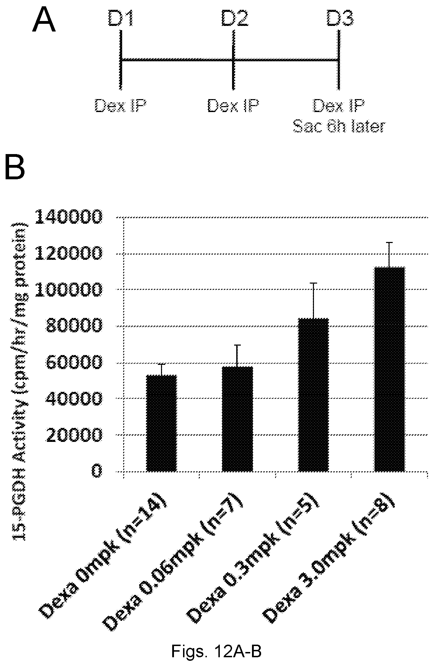

Fig. 12A shows a schema of near doubling of 15-PGDH enzyme activity in colons of dexamethasone treated mice. Fig. 12B shows findings that corticosteroids increase colon 15-PGDH activity suggesting that these agents paradoxically induce a negative feedback loop that would act to retard healing of colon mucosa in ulcerative colitis and intestinal mucosa in Crohn's disease. These findings predict that combining corticosteroid therapy with a 15-PGDH inhibitor would be predicted to improve the efficacy of corticosteroid therapy of ulcerative colitis and Crohn's disease.

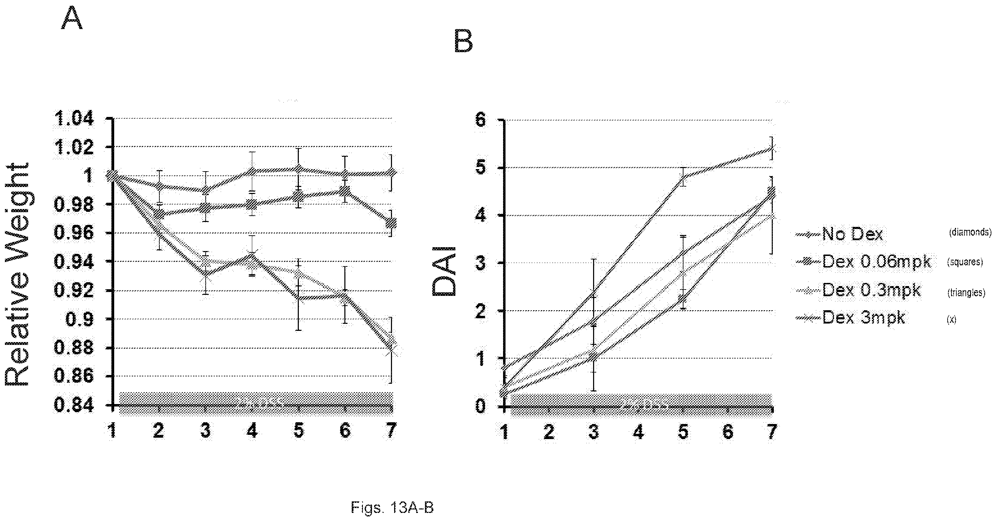

Figs. 13(A-B) show higher dexamethasone doses exacerbate colitis induction by DSS. 8-week old FVB/NJ male mice were exposed to 2% DSS in drinking water concurrent with daily dexamethasone intraperitoneal injections at specified dose, 0 mpk (diamonds), 0.06 mpk (squares), 0.3 mpk (triangles), 3.0 mpk(x). To compare the effects of increasing dexamethasone doses on the induction of colitis, daily weights and disease activity indices (severity of diarrhea and hematochezia) were compared and are graphed as shown (mean ± SEM, N=5-8), with relative daily weights shown in Fig. 13A and daily disease activity shown in Fig. 13B. Higher doses of dexamethasone significantly exacerbated the induction of colitis; mice with 0.3 or 3mpk of dexamethasone displayed significantly worse weight loss and mice with 3mpk developed significantly worse disease activity compared to the lower dose.

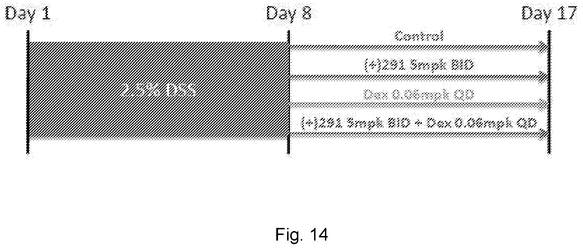

Fig. 14 shows the schematic of a study in which mice receive 7 days of 2.5% DSS in drinking water (from day 1 to day 8), a regime that induces murine colitis. Starting on day 8 mice are then treated with either: vehicle control; with a 15-PGDH inhibitor - (+) SW033291 at 5 mpk IP twice daily (abbreviated (+) 291); with dexamethasone 0.06 mpk IP daily (abbreviated dex); or with the combination of (+) SW033291 at 5 mpk IP twice daily plus dexamethasone 0.06 mpk IP daily. (mpk =mg/kg).

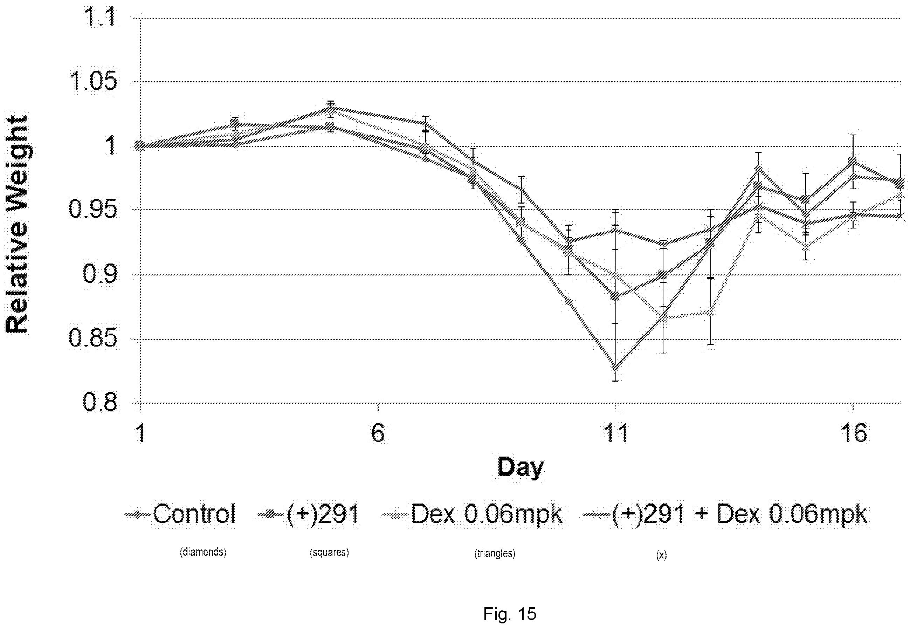

Fig. 15 shows daily weights of mice on the study from days 1-17, mice were treated with control (diamonds), SW033291 (squares), dexamethasone 0.06mpk (triangles), and combination of both SW033291 and dexamethasone (x). While both (+) SW033291 (squares) and dexamethasone (triangles) treatment as single agents provide some amelioration of weight loss, the combination of (+) SW033291 plus dexamethasone (x) was significantly more effective.

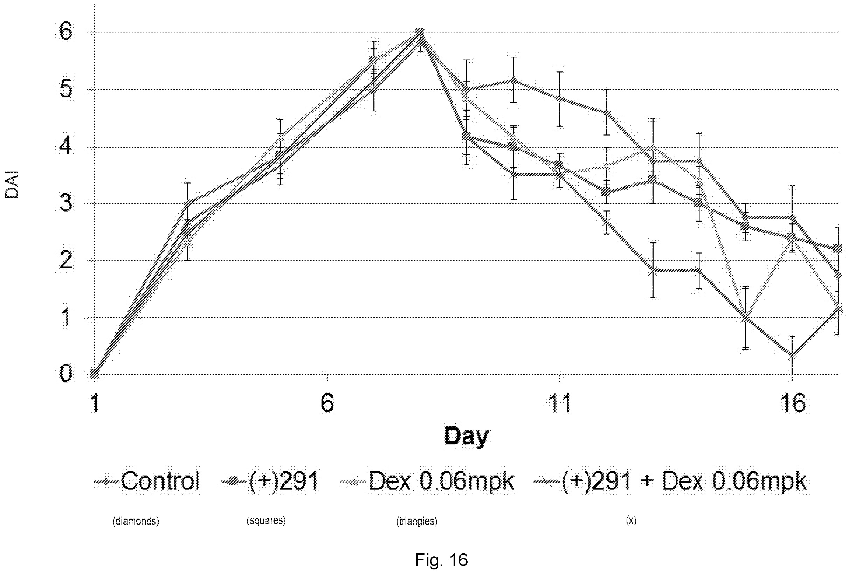

Fig. 16 shows disease activity as measured by the disease activity index (DAI) in which diarrhea (on a 0-3 scale) and fecal blood (on a 0-3 scale) are combined (on a 0-6 scale). Mice were treated with control (diamonds), SW033291 (squares), dexamethasone 0.06mpk (triangles), and combination of both SW033291 and dexamethasone (x). While both (+) SW033291 (squares) and dexamethasone (triangles) treatment as single agents provide some amelioration of DAI, the combination of (+) SW033291 plus dexamethasone (x) was significantly more effective.

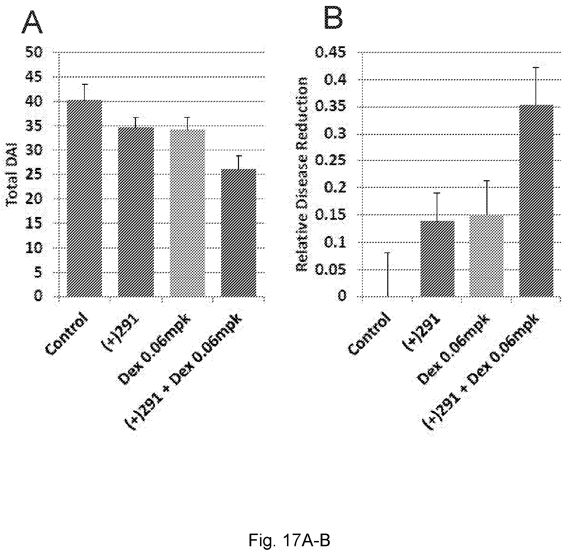

Fig. 17A graphs these results showing area under the DAI curve (total DAI) and Fig. 17B the percent decrease in total DAI (relative disease reduction) graphed. Single agent (+) SW033291 reduced total DAI by 14%. Single agent dexamethasone reduced total DAI by 15%. However the combination of (+) SW033291 plus dexamethasone reduced total DAI by 35%.

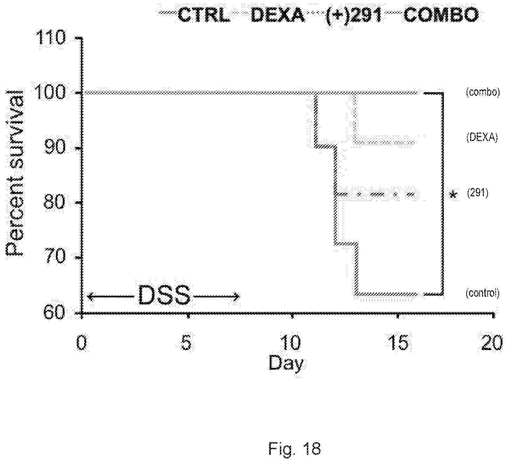

Fig. 18 graphs the survival of mice with control, dexamethasone, SW033291, and combination treatment on a daily basis through day 16 of the disease model (N=12 per group). *p<0.05 by Mantel-Cox test.

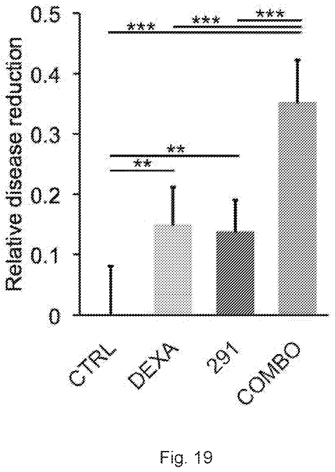

Fig. 19 is a regraphing of the data of Fig. 17B, with P-values, and a reordering of the sequence of presenting the groups. Combination group (green bar) is significantly improved compared to control (blue bar) or dexamethasone (yellow bar) or (+)- SW033291 (red bar) and is superior to either of the monotherapy regimens. Means + SEM (N=6 per arm). **p<0.01, ***p<0.005 by ANOVA and Student's t-test.

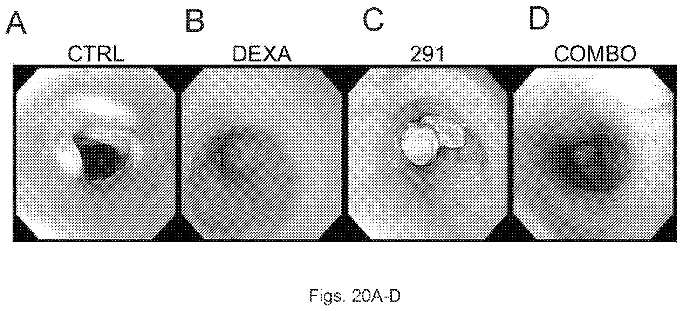

Figs. 20(A-D) show representative endoscopic images for each group (A) control, (B) dexamethasone, (C) SW033291, and (D) combination) on day 13 of treatment. Signs of mucosal bleeding and reduced wall transparency were evident in the control group whereas reduced wall transparency is more prominent in the dexamethasone group when compared to (+)-SW033291 or combination.

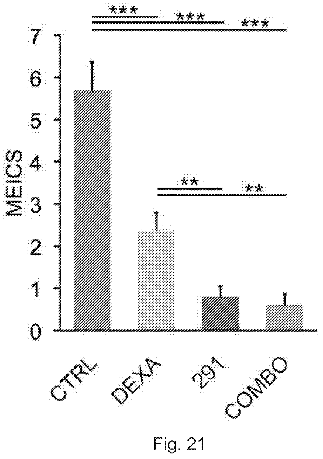

Fig. 21 shows graphs of murine endoscopic index of colitis severity (MEICS) scores as means + SEM (N=8-10 per arm) on day 13 for each treatment group (control, dexamethasone, SW033291, and combination). **p<0.01, ***p<0.005 by ANOVA and Student's t-test.

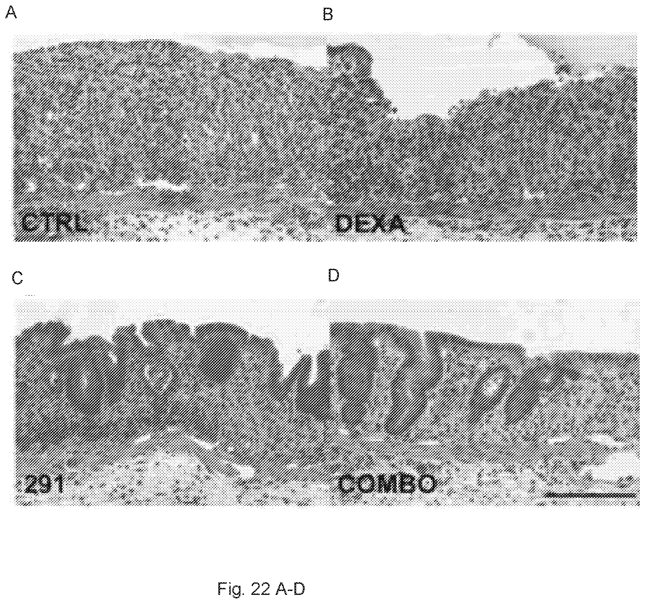

Figs. 22(A-D) show representative histological pictures of distal colons on day 13 of each treatment group (A) control, (B) dexamethasone, (C) SW033291, and (D) combination. Destruction of epithelial crypt structures were more severely manifested in both control and dexamethasone-treated mice compared to (+)-SW033291- or combination-treated mice. Scale bar: 200µm.

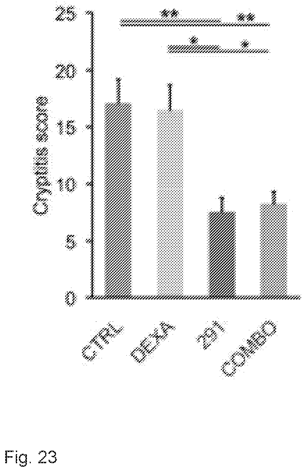

Fig. 23 shows graphs of semi-quantitatively scored histological extent of inflammatory damage to the crypts ("cryptits"). **p<0.01, ***p<0.005 by ANOVA and Student's t-test.

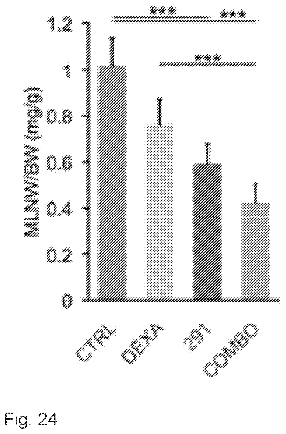

Fig. 24 shows graphs of the severity of mesenteric lymphadenopathy assessed by collective mesenteric lymph node weight normalized by body weight on day 13 of each treatment group (control, dexamethasone, SW033291, and combination). Means +SEM (N=11-16). ***p<0.005 by ANOVA and Student's t-test.

## Claims

1. A 15-PGDH inhibitor in combination with a corticosteroid for use in treating intestinal, gastrointestinal, or bowel disorders;
wherein the corticosteroid is selected from the group consisting of dexamethasone, dexamethasone acetate and dexamethasone sodium phosphate; and
wherein the 15-PGDH inhibitor includes the compound SW033291: 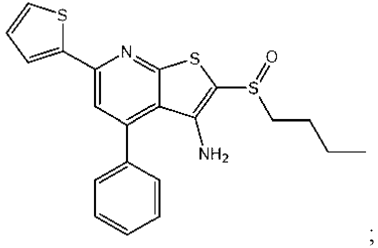
or a pharmaceutically acceptable salt thereof.

2. The 15-PGDH inhibitor in combination with a corticosteroid for use of claim 1, wherein the disorder comprises at least one of oral ulcers, gum disease, gastritis, colitis, ulcerative colitis, gastric ulcers, inflammatory bowel disease, and Crohn's disease.

3. The 15-PGDH inhibitor in combination with a corticosteroid for use of claim 1, wherein the disease is inflammatory bowel disease.

4. The 15-PGDH inhibitor in combination with a corticosteroid for use of any of claims 1 to 3, wherein the corticosteroid induces 15-PGDH expression.

5. The 15-PGDH inhibitor in combination with a corticosteroid for use of any of claims 1 to 4, wherein the 15-PGDH inhibitor is effective to attenuate corticosteroid induced adverse and/or cytotoxic effects in a subject, or to increase therapeutic efficacy.

6. The 15-PGDH inhibitor in combination with a corticosteroid for use of any of claims 1 to 5, wherein the disorder comprises inflammation of the esophagus, inflammation of the glottis, inflammation of the epiglottis, inflammation of the tonsils, inflammation of the oropharynx, eosinophilic esophagitis, gastroesophageal reflux disease (GERD), non-erosive reflux disease (NERD), erosive esophagitis, Barrett's esophagus, eosinophilic gastroenteritis, hypereosinophilic syndrome, corrosive (caustic) chemical esophagitis, radiation-induced esophagitis, chemotherapy-induced esophagitis, transient drug-induced esophagitis, persistent drug-induced esophagitis, Crohn's disease of the esophagus, and pseudomembranous esophagitis.

7. The 15-PGDH inhibitor in combination with a corticosteroid for use of any of claims 1 to 5, wherein the corticosteroid is dexamethasone.

## Patentansprüche

1. Ein 15-PGDH-Inhibitor in Kombination mit einem Kortikosteroid zur Verwendung bei der Behandlung von Darm-, Magen-Darm- oder Verdauungsstörungen;
wobei das Kortikosteroid aus der Gruppe ausgewählt ist, die aus Dexamethason, Dexamethasonacetat und Dexamethason-Natriumphosphat besteht; und
wobei der 15-PGDH-Inhibitor die Verbindung SW033291 umfasst: 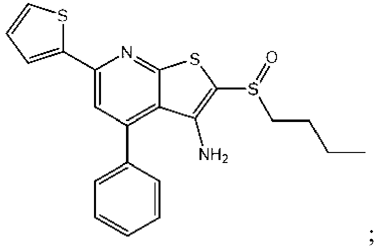
oder ein pharmazeutisch verträgliches Salz davon.

2. Der 15-PGDH-Inhibitor in Kombination mit einem Kortikosteroid zur Verwendung nach Anspruch 1, wobei die Erkrankung mindestens eine der folgenden umfasst:
Mundgeschwüre, Zahnfleischerkrankungen, Gastritis, Kolitis, Colitis ulcerosa, Magengeschwüre, entzündliche Darmerkrankungen und Morbus Crohn.

3. Der 15-PGDH-Inhibitor in Kombination mit einem Kortikosteroid zur Verwendung nach Anspruch 1, wobei es sich bei der Erkrankung um eine entzündliche Darmerkrankung handelt.

4. Der 15-PGDH-Inhibitor in Kombination mit einem Kortikosteroid zur Verwendung gemäß einem der Ansprüche 1 bis 3, wobei das Kortikosteroid die 15-PGDH-Expression induziert.

5. Der 15-PGDH-Inhibitor in Kombination mit einem Kortikosteroid zur Verwendung gemäß einem der Ansprüche 1 bis 4, wobei der 15-PGDH-Inhibitor wirksam ist, um durch das Kortikosteroid induzierte Nebenwirkungen und/oder zytotoxische Wirkungen bei einem Patienten abzuschwächen oder die therapeutische Wirksamkeit zu erhöhen.

6. Der 15-PGDH-Inhibitor in Kombination mit einem Kortikosteroid zur Verwendung gemäß einem der Ansprüche 1 bis 5, wobei die Erkrankung eine Entzündung der Speiseröhre, eine Entzündung der Stimmritze, eine Entzündung der Epiglottis, eine Entzündung der Mandeln, eine Entzündung des Oropharynx, eine eosinophile Ösophagitis, gastroösophageale Refluxkrankheit (GERD), nicht-erosive Refluxkrankheit (NERD), erosive Ösophagitis, Barrett-Ösophagus, eosinophile Gastroenteritis, hypereosinophiles Syndrom, korrosive (ätzende) chemische Ösophagitis, strahleninduzierte Ösophagitis, chemotherapieinduzierte Ösophagitis, vorübergehende arzneimittelinduzierte Ösophagitis, persistierende arzneimittelinduzierte Ösophagitis, Morbus Crohn der Speiseröhre und pseudomembranöse Ösophagitis umfasst.

7. Der 15-PGDH-Inhibitor in Kombination mit einem Kortikosteroid zur Verwendung gemäß einem der Ansprüche 1 bis 5, wobei das Kortikosteroid Dexamethason ist.

## Revendications

1. Inhibiteur de 15-PGDH en combinaison avec un corticostéroïde pour une utilisation dans le traitement de troubles intestinaux, gastro-intestinaux ou de l'intestin ;
dans lequel le corticostéroïde est sélectionné dans le groupe consistant en dexaméthasone, acétate de dexaméthasone et phosphate sodique de dexaméthasone ; et
dans lequel l'inhibiteur de 15-PGDH comporte le composé SW033291 : 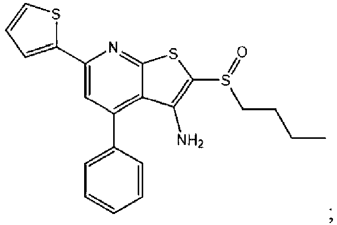
ou un sel pharmaceutiquement acceptable de celui-ci.

2. Inhibiteur de 15-PGDH en combinaison avec un corticostéroïde pour une utilisation selon la revendication 1, dans lequel le trouble comprend au moins un parmi ulcères buccaux, maladie gingivale, gastrite, colite, rectocolite hémorragique, ulcères gastriques, maladie inflammatoire de l'intestin et maladie de Crohn.

3. Inhibiteur de 15-PGDH en combinaison avec un corticostéroïde pour une utilisation selon la revendication 1, dans lequel la maladie est une maladie inflammatoire de l'intestin.

4. Inhibiteur de 15-PGDH en combinaison avec un corticostéroïde pour une utilisation selon l'une quelconque des revendications 1 à 3, dans lequel le corticostéroïde induit une expression de 15-PGDH.

5. Inhibiteur de 15-PGDH en combinaison avec un corticostéroïde pour une utilisation selon l'une quelconque des revendications 1 à 4, dans lequel l'inhibiteur de 15-PGDH est efficace pour atténuer des effets néfastes et/ou cytotoxiques induits par corticostéroïde chez un sujet, ou pour augmenter une efficacité thérapeutique.

6. Inhibiteur de 15-PGDH en combinaison avec un corticostéroïde pour une utilisation selon l'une quelconque des revendications 1 à 5, dans lequel le trouble comprend une inflammation de l'œsophage, inflammation de la glotte, inflammation de l'épiglotte, inflammation des amygdales, inflammation de l'oropharynx, œsophagite à éosinophiles, reflux gastro-œsophagienne (RGO), maladie par reflux non érosif (MRNE), œsophagite érosive, œsophage de Barrett, gastro-entérite à éosinophiles, syndrome hyperéosinophilique, œsophagite chimique corrosive (caustique), œsophagite induite par rayonnement, œsophagite induite par chimiothérapie, œsophagite transitoire induite par médicament, œsophagite persistante induite par médicament, maladie de Crohn de l'œsophage et œsophagite pseudomembraneuse.

7. Inhibiteur de 15-PGDH en combinaison avec un corticostéroïde pour une utilisation selon l'une quelconque des revendications 1 à 5, dans lequel le corticostéroïde est la dexaméthasone.
